# EUROPEAN PATENT APPLICATION

(11) **EP 3 909 602 A1**
(43) Date of publication of application: **17.11.2021**
(21) Application number: 21151619.0
(22) Date of filing: 24.04.2015
(51) Int. Cl.: A61K 39/00, A61K 48/00, C12N 15/00, A61K 31/44, A61K 31/436, A61K 38/47, A61K 39/395, C07K 16/06, C07K 16/28

(54) **METHODS OF PERMITTING A SUBJECT TO RECEIVE MULTIPLE DOSES OF RECOMBINANT ADENO-ASSOCIATED VIRUS**

(30) Priority: 25.04.2014 US 201461984607 P
(62) Divisional of application: 15782460.8
(71) Applicant: University of Florida Research Foundation, Inc., Gainesville, FL 32611 (US)
(72) Inventor: BYRNE, Barry John, Gainesville, FL 32607 (US); FALK, Darin J., Gainesville, FL 32653-0710 (US); CORTI, Manuela, Alachua, FL 32615-9355 (US)
(74) Representative: Potter Clarkson

(57) **Abstract**

Provided herein are compositions, kits and methods related to permitting a human or non-human primate subject to receive multiple doses of recombinant adeno-associate virus (rAAV) vectors. In some aspects, provided herein are methods comprising administering an anti-CD20 antibody, optionally in combination with an mTOR inhibitor, to the subject to permit multiple doses of an rAAV vector to be administered.

SEQUENCE LISTING

<110> University of Florida Research Foundation, Inc.

<120> METHODS OF PERMITTING A SUBJECT TO RECEIVE MULTIPLE DOSES OF
      RECOMBINANT ADENO-ASSOCIATED VIRUS

<130> WOLBT/P62056EP

<140> EP 15782460.8
<141> 2015-04-24

<150> PCT/US2015/027538
<151> 2015-04-24

<150> US 61/984,607
<151> 2014-04-25

<160> 1

<170> PatentIn version 3.5

<210> 1
<211> 297
<212> PRT
<213> Homo sapiens

<400> 1

Met Thr Thr Pro Arg Asn Ser Val Asn Gly Thr Phe Pro Ala Glu Pro
1               5                   10                  15

Met Lys Gly Pro Ile Ala Met Gln Ser Gly Pro Lys Pro Leu Phe Arg
            20                  25                  30

Arg Met Ser Ser Leu Val Gly Pro Thr Gln Ser Phe Phe Met Arg Glu
        35                  40                  45

Ser Lys Thr Leu Gly Ala Val Gln Ile Met Asn Gly Leu Phe His Ile
    50                  55                  60

Ala Leu Gly Gly Leu Leu Met Ile Pro Ala Gly Ile Tyr Ala Pro Ile
65                  70                  75                  80

Cys Val Thr Val Trp Tyr Pro Leu Trp Gly Gly Ile Met Tyr Ile Ile
                85                  90                  95

Ser Gly Ser Leu Leu Ala Ala Thr Glu Lys Asn Ser Arg Lys Cys Leu
            100                 105                 110

Val Lys Gly Lys Met Ile Met Asn Ser Leu Ser Leu Phe Ala Ala Ile
        115                 120                 125

Ser Gly Met Ile Leu Ser Ile Met Asp Ile Leu Asn Ile Lys Ile Ser
    130                 135                 140

His Phe Leu Lys Met Glu Ser Leu Asn Phe Ile Arg Ala His Thr Pro
145                 150                 155                 160

Tyr Ile Asn Ile Tyr Asn Cys Glu Pro Ala Asn Pro Ser Glu Lys Asn
                165                 170                 175

Ser Pro Ser Thr Gln Tyr Cys Tyr Ser Ile Gln Ser Leu Phe Leu Gly
            180                 185                 190

Ile Leu Ser Val Met Leu Ile Phe Ala Phe Phe Gln Glu Leu Val Ile
        195                 200                 205

Ala Gly Ile Val Glu Asn Glu Trp Lys Arg Thr Cys Ser Arg Pro Lys
    210                 215                 220

Ser Asn Ile Val Leu Leu Ser Ala Glu Glu Lys Lys Glu Gln Thr Ile
225                 230                 235                 240

Glu Ile Lys Glu Glu Val Val Gly Leu Thr Glu Thr Ser Ser Gln Pro
                245                 250                 255

Lys Asn Glu Glu Asp Ile Glu Ile Ile Pro Ile Gln Glu Glu Glu Glu
            260                 265                 270

Glu Glu Thr Glu Thr Asn Phe Pro Glu Pro Pro Gln Asp Gln Glu Ser
        275                 280                 285

Ser Pro Ile Glu Asn Asp Ser Ser Pro
    290                 295

## Description

### CROSS-REFERENCE TO RELATED APPLICATIONS

This application claims the benefit under 35 U.S.C. § 119(e) of U.S. provisional application number 61/984,607, filed April 25, 2014, which is incorporated by reference herein in its entirety.

### GOVERNMENT SUPPORT

This invention was made with government support under HL59412 awarded by the National Institutes of Health. The government has certain rights in the invention.

### BACKGROUND OF THE INVENTION

Immune responses to recombinant adeno-associate virus (rAAV) vectors and gene products currently prevent safe and effective administration of more than one dose of a rAAV vector to a subject more than one time points. There is currently no method available that permits administration of multiple doses of rAAV vectors to a subject over multiple time points.

### SUMMARY OF THE INVENTION

Aspects of the disclosure relate to methods of permitting a subject to receive multiple doses of a rAAV vector. In some embodiments, B cell depletion using one or more pharmaceutical agents may be utilized to permit the subject to receive multiple doses of a rAAV vector. In some embodiments, B cell depletion using one or more pharmaceutical agents may be utilized to permit a subject who has received a prior administration of a rAAV vector to receive a subsequent administration of the same or a different rAAV vector.

The disclosure is premised, in part, on a study showing that a subject who had been B-cell depleted did not have an immune response to the capsid or transgene product of a recombinant adeno-associated virus (rAAV) vector that was administered after the B-cell depletion. These results suggest that B-cell depletion inhibits the immune response to the rAAV vector and its products, allowing for multiple administrations of a rAAV vector to be given to a single subject at multiple time points.

Accordingly, aspects of the disclosure relate to methods of permitting a subject to receive multiple doses of a rAAV vector.

Some aspects of the disclosure relate to a method of permitting a human or non-human primate subject to receive multiple doses of a recombinant adeno-associate virus (rAAV) vector. In some embodiments, the method comprises administering an anti-CD20 antibody and a subsequent rAAV vector to a human or non-human primate subject that has previously been administered a prior rAAV vector. In some embodiments, the method comprises administering a subsequent rAAV vector to a human or non-human primate subject that has previously been administered a prior rAAV vector and an anti-CD20 antibody. In some embodiments, the subsequent and prior rAAV vectors are the same serotype.

In some embodiments, the anti-CD20 antibody is administered more than once. In some embodiments, the anti-CD20 antibody is administered at least once prior to the administration of the subsequent rAAV vector.

In some embodiments, the method further comprises administering the prior rAAV vector to the subject prior to the administration of the subsequent rAAV vector. In some embodiments, the anti-CD20 antibody is administered more than once and wherein the anti-CD20 antibody is administered to the subject at least once prior to the administration of the subsequent rAAV vector and at least once prior to the administration of the prior rAAV vector.

In some embodiments, the method further comprises administering to the subject an mTOR pathway inhibitor. In some embodiments, the mTOR pathway inhibitor is rapamycin. In some embodiments, the anti-CD20 antibody and the mTOR pathway inhibitor are each administered more than once. In some embodiments, the anti-CD20 antibody and the mTOR pathway inhibitor are each administered at least once prior to the administration of the subsequent rAAV vector.

In some embodiments, the method further comprises administering the prior rAAV vector to the subject prior to the administration of the subsequent rAAV vector. In some embodiments, the anti-CD20 antibody and the mTOR pathway inhibitor are each administered more than once and wherein the anti-CD20 antibody and the mTOR pathway inhibitor are each administered to the subject at least once prior to the administration of the subsequent rAAV vector and at least once prior to the administration of the first rAAV vector.

In some embodiments, the anti-CD20 antibody is rituximab. In some embodiments, the subject is a human.

In some embodiments, the administration of the prior rAAV vector to the subject occurs at least one day prior to the administration of the subsequent rAAV vector. In some embodiments, the administration of the prior rAAV vector to the subject occurs at least one week prior to the administration of the subsequent rAAV vector. In some embodiments, the administration of the prior rAAV vector to the subject occurs at least one month prior to the administration of the subsequent rAAV vector. In some embodiments, the administration of the prior rAAV vector to the subject occurs at least one year prior to the administration of the subsequent rAAV vector.

The details of one or more embodiments of the disclosure are set forth in the description below. Other features or advantages of the present disclosure will be apparent from the following drawings and detailed description of several embodiments, and also from the appending claims.

### BRIEF DESCRIPTION OF THE DRAWINGS

The following drawings form part of the present specification and are included to further demonstrate certain aspects of the present disclosure, which can be better understood by reference to one or more of these drawings in combination with the detailed description of specific embodiments presented herein.

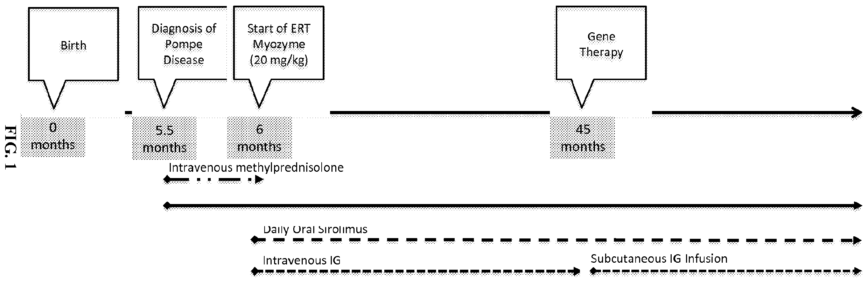

FIG. 1 is a schematic showing an exemplary dosage regimen of an infant with Pompe disease who received rituximab and sirolimus, followed by subsequent gene therapy. Immediately after diagnosis with Pompe, the subject received enzyme replacement therapy (ERT) and immune modulation for management of antibody response as part of treatment with ERT. At 45 months of age, the patient received rAAV1-GAA intramuscular gene transfer to the diaphragm;

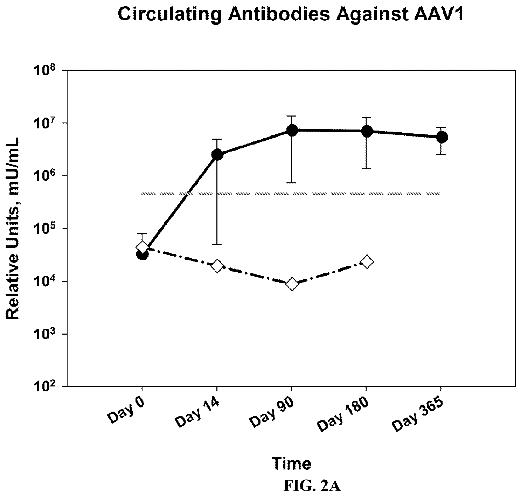

FIG. 2A is a graph showing exemplary circulating antibodies over time against rAAV1 in a 3.5 year-old subject with Pompe disease (dash-dot lines, diamonds) who was treated with rituximab and sirolimus prior to treatment with rAAV1-GAA versus five control subjects with Pompe disease (solid lines, circles), age 2.5 to 15 years, who received rAAV1-GAA but no B-cell depletion, where the light horizontal lines represent +2 SD population level and day 0 on the x-axis is the day each subject received gene therapy;

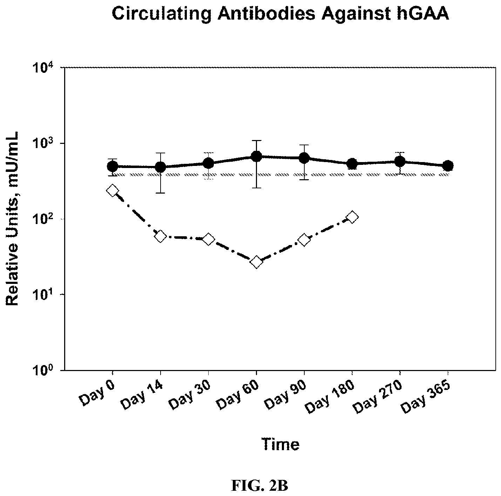

FIG. 2B is a graph showing exemplary circulating antibodies against human acid alpha-glucosidase (GAA) in a 3.5 year-old subject with Pompe disease (dash-dot lines, diamonds) who was treated with rituximab and sirolimus prior to treatment with rAAV1-GAA versus five control subjects with Pompe disease (solid lines, circles), age 2.5 to 15 years, who recevied rAAV1- GAA but no B-cell depletion, where the light horizontal lines represent +2 SD population level and day 0 on the x-axis is the day each subject received gene therapy;

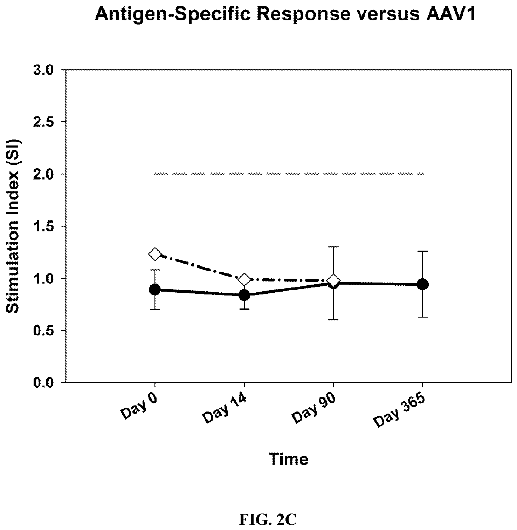

FIG. 2C is a graph showing an exemplary antigen-specific response to AAV1 in a 3.5 year-old subject with Pompe disease (dash-dot lines, diamonds) who was treated with rituximab and sirolimus prior to treatment with rAAV1-GAA versus five control subjects with Pompe disease (solid lines, circles), age 2.5 to 15 years, who recevied rAAV1-GAA but no B-cell depletion, where the light horizontal lines represent +2 SD population level and day 0 on the x-axis is the day each subject received gene therapy;

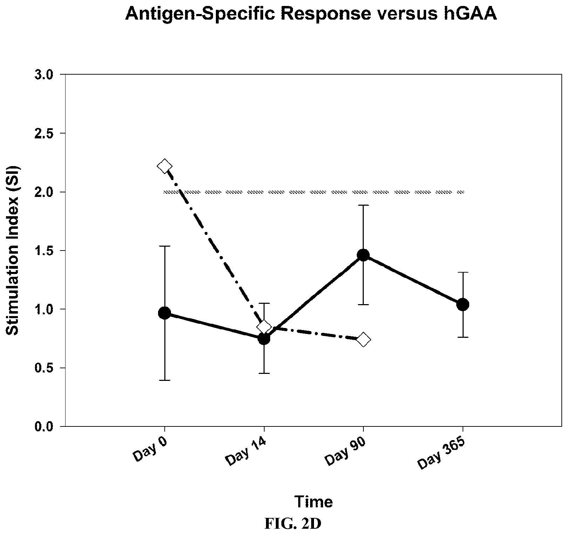

FIG. 2D is a graph showing an exemplary antigen-specific response to human acid alpha-glucosidase (GAA) protein in a 3.5 year-old subject with Pompe disease (dash-dot lines, diamonds) who was treated with rituximab and sirolimus prior to treatment with rAAV1-GAA versus five control subjects with Pompe disease (solid lines, circles), age 2.5 to 15 years, who recevied rAAV1- GAA but no B-cell depletion, where the light horizontal lines represent +2 SD population level and day 0 the day each subject received gene therapy;

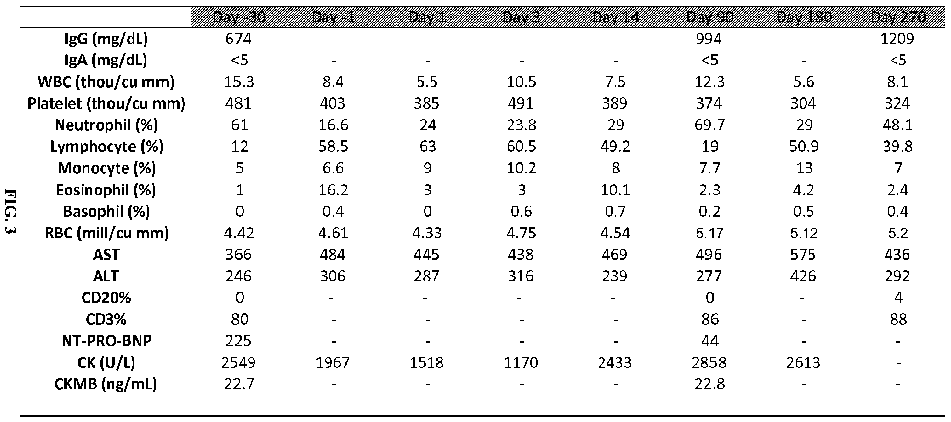

FIG. 3 is a table summarizing exemplary laboratory safety data for a subject with Pompe disease treated with rituximab and sirolimus followed by rAAV1-GAA gene therapy;

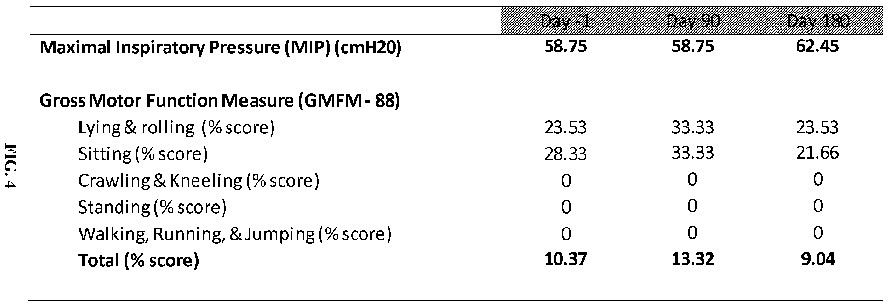

FIG. 4 is a table summarizing exemplary functional data, such a maximal inspiratory pressure and motor function measure, for a subject with Pompe disease treated with rituximab and sirolimus followed by rAAV1-GAA gene therapy; and

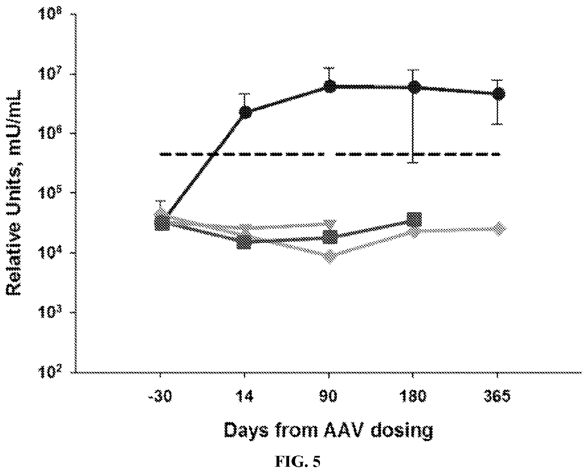

FIG. 5 is a graph showing the circulating antibodies against AAV1 in subjects treated with rAAV1-CMV-GAA without immunosuppression (line with circles) and in subjects treated with rAAV1-CMV-GAA with immunosuppression (lines with triangles, diamonds, and squares). The dotted line shows the +2 standard deviation (SD) population mean.

### DETAILED DESCRIPTION OF THE INVENTION

Immune responses triggered by the first administration of a recombinant adeno-associated viral (rAAV) vector to a subject, and to any subsequent administrations, represent a major obstacle for successful gene transfer. Anti-capsid and anti-transgene humoral and cell-mediated responses have been previously observed in all preclinical models and human subjects who received gene therapy for congenital myopathies. Such immune responses may result in reduced efficacy of the gene transfer over time and/or may preclude the possibility of re-administration of the same vector or administration of other rAAV vectors to the same subj ect.

Gene therapy strategies may also require repeat administrations of rAAV vectors as a result of several limitations inherent to clinical design. For example, administration of doses below therapeutic efficacy in patients enrolled in early phase clinical trials may require subsequent administration of higher doses to the same patients to obtain therapeutic efficacy. In another example, progressive reduction of the therapeutic gene expression could result over time in response to an increase in mass in patients treated at a young age.

As a result, there is a need for methods that permit a subject to receive multiple doses of rAAV vectors. As described herein, a patient having Pompe disease (a lysosomal storage disease) dosed with a rAAV1-GAA vector after receiving rituximab and sirolimus was assessed for immune responses to both the rAAV capsid and the transgene. It was observed that B-cell ablation with rituximab prior to rAAV vector exposure resulted in nonresponsiveness to both capsid and transgene, therefore allowing for the possibility of repeat administrations of the same vector or a different vector in the future. The data provided herein establishes a clinically relevant approach to blocking immune responses to rAAV.

Accordingly, provided herein are methods and kits related to permitting a subject to receive multiple doses of rAAV vectors including multiple doses of the same vector serotype.

### Methods

Aspects of the disclosure relate to methods of permitting a human or non-human primate subject to receive multiple doses of a recombinant adeno-associate virus (rAAV) vector as described herein.

In some embodiments, the method comprises administering a B cell depletion treatment and a subsequent rAAV vector as described herein to the human or non-human primate subject that has previously been administered a prior rAAV vector as described herein. In some embodiments, the method comprises administering an anti-CD20 antibody as described herein (e.g., rituximab) and a subsequent rAAV vector as described herein to a human or non-human primate subject that has previously been administered a prior rAAV vector as described herein. In some embodiments, the method further comprises administering an mTOR pathway inhibitor as described herein.

In some embodiments, the method comprises administering a B cell depletion treatment and a subsequent rAAV vector as described herein to a human or non-human primate subject that has previously been administered a B cell depletion treatment and a prior rAAV vector as described herein. In some embodiments, the method comprises administering an anti-CD20 antibody as described herein (e.g., rituximab) and a subsequent rAAV vector as described herein to a human or non-human primate subject that has previously been administered an anti-CD20 antibody as described herein and a prior rAAV vector as described herein. In some embodiments, the method further comprises administering an mTOR pathway inhibitor as described herein.

In some embodiments, the method comprises administering a subsequent rAAV vector as described herein to a human or non-human primate subject that has previously been administered a B cell depletion treatment and a prior rAAV vector as described herein. In some embodiments, the method comprises a subsequent rAAV vector as described herein to a human or non-human primate subject that has previously been administered an anti-CD20 antibody as described herein and a prior rAAV vector as described herein. In some embodiments, the method further comprises administering an mTOR pathway inhibitor as described herein.

As used herein, a subsequent rAAV vector is a rAAV vector that is administered temporally after the prior rAAV vector. For example, the prior rAAV is administered at a first time point and the subsequent rAAV vector is administered at a second time point, where the first time point occurs prior to the second time point (e.g., by at least one day, at least one week, at least one month, or at least one year). In some embodiments, the first time point occurs at a first visit to a physician and the second time point occurs at a second visit to a physician (e.g., at least one day, at least one week, at least one month, or at least one year later than the first visit). The subsequent rAAV vector may be, for example, (a) a second administration of a rAAV vector following a prior first administration of a rAAV vector to the subject; (b) a third administration of a rAAV vector following a prior first administration of a rAAV vector and a prior second administration to the subject; (c) a fourth administration of a rAAV vector following a prior first administration of a rAAV vector, a prior second administration to the subject and a prior third administration to the subject; (d) a fifth administration of a rAAV vector following a prior first administration of a rAAV vector, a prior second administration to the subject, a prior third administration to the subject and a prior fourth administration to the subject or (e) a sixth, seventh, eighth, ninth, tenth, or more administrations where the subject has received five, six, seven, eight, nine, or more prior administrations, respectively. In some embodiments, the subsequent rAAV vector is administered at a later time point (e.g., at least one day later, at least one month later, at least one year later, at least 5 years later, at least 10 years later) than at least one prior administration of a rAAV vector to the subject. In some embodiments, a further rAAV vector is administered after the subsequent rAAV vector, meaning that the subsequent rAAV vector may be a middle administration between one or more prior administrations of a rAAV vector and one or more further administrations of a rAAV vector. In some embodiments, the prior rAAV vector is the first administration of a rAAV vector that a subject has received in their lifetime (i.e., the subject has not received a rAAV vector administration prior to the prior rAAV vector). In some embodiments the prior rAAV vector is a second, third, fourth, etc. administration of a rAAV vector to a subject (i.e., the subject has received a rAAV vector administration prior to the prior rAAV vector) and subsequent rAAV vector is an administration of a rAAV vector that occurs after the administration of the prior rAAV vector.

Accordingly, in some embodiments a human or non-human primate is treated with a series of rAAV vector administrations (e.g., 2, 3, 4, 5, 6, 7, 8, 9, 10, or more) at different time points (e.g., each separated by at least a day, week, month, year, or longer), wherein each administration comprises an rAAV vector being administered in the context of a B-cell depletion treatment as described herein. It should be appreciated that in some embodiments, the rAAV vector being administered at each time point is the same rAAV serotype. However, in some embodiments different serotypes may be used at different time points. It also should be appreciated that for different time points the rAAV vector that is being administered may include different nucleic acid sequences encoding different genes or different versions of the same gene. It also should be appreciated that in some embodiments, the same B-cell depletion treatment may be used for one or more different time points. However, in some embodiments different B-cell depletion treatments may be used for one or more different time points.

In some embodiments, one or more doses of the anti-CD20 antibody and/or the mTOR pathway inhibitor are provided in an amount and at a frequency that suppresses or inhibits the B-cell response and/or depletes the number of B-cells sufficiently to avoid or reduce an immune response to a subsequent rAAV vector in a subject that has received a prior rAAV vector. In some embodiments, the anti-CD20 antibody and/or the mTOR pathway inhibitor are administered to the subject more than once (e.g., 2, 3, 4, 5, 6, 7, 8, 9, 10, 15, 20, 30 or more times). In some embodiments, the anti-CD20 antibody and/or the mTOR pathway inhibitor are administered at least once (e.g., 1, 2, 3, 4, 5, 6, 7, 8, 9, 10, 15, 20, 30 or more times) prior to the administration of the subsequent rAAV vector. In some embodiments, the anti-CD20 antibody and/or the mTOR pathway inhibitor are administered at least once (e.g., 1, 2, 3, 4, 5, 6, 7, 8, 9, 10, 15, 20, 30 or more times) prior to the administration of the subsequent rAAV vector and at least once (e.g., 1, 2, 3, 4, 5, 6, 7, 8, 9, 10, 15, 20, 30 or more times) after administration of the subsequent rAAV vector. In some embodiments, the anti-CD20 antibody and/or the mTOR pathway inhibitor are administered concurrently with the subsequent rAAV vector. In some embodiments, the anti-CD20 antibody and/or the mTOR pathway inhibitor are administered prior to and concurrently with the subsequent rAAV vector. It is to be understood that the anti-CD20 antibody and the mTOR pathway inhibitor can be administered at the same time or at different times and using the same dosage schedule or different dosage schedules (e.g., the anti-CD20 antibody may be administered weekly while the mTOR pathway inhibitor may be administered daily). It is also to be understood that the anti-CD20 antibody and the mTOR pathway inhibitor can be administered a different number of times, such that the number of administrations of the anti-CD20 antibody and the mTOR pathway inhibitor are different. For example, the anti-CD20 antibody can be administered at least once (e.g., 1, 2, 3, 4, 5, 6, 7, 8, 9, 10, 15, 20, 30 or more times) and the mTOR pathway inhibitor can be administered at least once (e.g., 1, 2, 3, 4, 5, 6, 7, 8, 9, 10, 15, 20, 30 or more times), wherein the number of administrations of the anti-CD20 antibody differs from the number of administrations of the mTOR pathway inhibitor.

In some embodiments, the method further comprises administering the prior rAAV vector described herein to the subject prior to the administration of the subsequent rAAV vector. In some embodiments, the anti-CD20 antibody and/or the mTOR pathway inhibitor are administered at least once (e.g., 1, 2, 3, 4, 5, 6, 7, 8, 9, 10, 15, 20, 30 or more times) prior to the administration of the subsequent rAAV vector and at least once (e.g., 1, 2, 3, 4, 5, 6, 7, 8, 9, 10, 15, 20, 30 or more times) prior to the administration of the prior rAAV vector. In some embodiments, the anti-CD20 antibody and/or the mTOR pathway inhibitor are administered at least once (e.g., 1, 2, 3, 4, 5, 6, 7, 8, 9, 10, 15, 20, 30 or more times) prior to the administration of the subsequent rAAV vector and at least once (e.g., 1, 2, 3, 4, 5, 6, 7, 8, 9, 10, 15, 20, 30 or more times) after administration of the subsequent rAAV vector as well as at least once (e.g., 1, 2, 3, 4, 5, 6, 7, 8, 9, 10, 15, 20, 30 or more times) prior to the administration of the prior rAAV vector and at least once (e.g., 1, 2, 3, 4, 5, 6, 7, 8, 9, 10, 15, 20, 30 or more times) after administration of the prior rAAV vector. In some embodiments, the anti-CD20 antibody and/or the mTOR pathway inhibitor are administered concurrently with the subsequent rAAV vector and concurrently with the prior rAAV vector. In some embodiments, the anti-CD20 antibody and/or the mTOR pathway inhibitor are administered prior to and concurrently with the subsequent rAAV vector and prior to and concurrently with the prior rAAV vector. In some embodiments, concurrent administration comprises administering a rAAV vector at approximately the same time as an anti-CD20 antibody and/or an mTOR pathway inhibitor. In some embodiments, a rAAV vector is administered with a dosage regimen that overlaps in part or in whole (e.g., at least one day, one week, one month, or more, of overlap) with a dosage regimen of an anti-CD20 antibody and/or an mTOR pathway inhibitor.

In some embodiments, the anti-CD20 antibody is administered to the subject at least once a week, at least once every 4 weeks, at least once every 8 weeks, at least once every 12 weeks, or any interval in between. In some embodiments, the anti-CD20 antibody is administered to the subject at least once every 12 weeks. In some embodiments, the mTOR pathway inhibitor is administered to the subject at least once a day, at least once a week, at least once a month, or any interval in between. In some embodiments, the mTOR pathway inhibitor is administered to the subject at least once a day.

In some embodiments, a method described herein may further comprise administering intravenous immunoglobulin (IVIG) to the subject. In some embodiments, the IVIG is administered to the subject at least once a week, at least once every 2 weeks, at least once every 3 weeks, at least once every 4 weeks, at least once every 8 weeks, at least once every 12 weeks, or any interval in between. In some embodiments, the IVIG is administered to the subject at least once every 4 weeks.

In some embodiments, the prior rAAV vector is administered to the subject at least one day (e.g., at least 1, 2, 3, 4, 5, 6, or 7 days), at least one week (e.g., at least 1, 2, 3, 4, 5, or 6 weeks), at least one month (e.g., at least 1, 2, 3, 4, 5, 6, 7, 8, 9, 10, 11, or 12 months), or at least one year (e.g., at least 1, 2, 3, 4, 5, 6, 7, 8, 9, 10, 11, 12, 13, 14, 15, 16, 17, 18, 19, or 20 years) prior to the administration of the subsequent rAAV vector.

In some embodiments, aspects of the disclosure relate to administering an anti-CD20 antibody and/or an mTOR pathway inhibitor at least once (e.g., 1, 2, 3, 4, 5, 6, 7, 8, 9, 10, 15, 20, 30 or more times) prior to, concurrently with, and/or after the administration of an rAAV vector to a subject who previously has received at least one treatment with (e.g., at least one dosage of) an enzyme replacement therapy. In some embodiments, a subject being treated with an enzyme replacement therapy and being transitioned to an rAAV-based therapy is treated with an anti-CD20 antibody and/or an mTOR pathway inhibitor at least once (e.g., 1, 2, 3, 4, 5, 6, 7, 8, 9, 10, 15, 20, 30 or more times) prior to, concurrently with, and/or after receiving a dose of an enzyme replacement therapy and prior to a first dose of rAAV (e.g., using an rAAV that delivers a gene encoding the same enzyme that was being administered during the enzyme replacement therapy. In some embodiments, an rAAV vector is administered with a dosage regimen that overlaps in part or in whole (e.g., at least one day, one week, one month, or more, of overlap) with a dosage regimen of an anti-CD20 antibody and/or an mTOR pathway inhibitor.

Compositions for use in a method described herein or compositions for manufacture of a medicament for use in a method described herein are also contemplated. In some embodiments, a composition comprising an anti-CD20 antibody for use in a method described herein is provided. In some embodiments, a composition comprising an anti-CD20 antibody and an mTOR pathway inhibitor for use in a method described herein is provided.

### Recombinant Adeno-Associated Virus (rAAV) vectors

Aspects of the disclosure relate to recombinant adeno-associated virus (rAAV) vectors for delivery of one or more heterologous nucleic acid sequences encoding a protein or polypeptide of interest into various tissues, organs, and/or cells.

The wild-type AAV genome is a single-stranded deoxyribonucleic acid (ssDNA), either positive- or negative-sensed. The genome comprises inverted terminal repeats (ITRs) at both ends of the DNA strand, and two open reading frames (ORFs): *rep* and *cap.* The *rep* ORF comprises four overlapping genes encoding Rep proteins required for the AAV life cycle. The *cap* ORF comprises overlapping genes encoding capsid proteins: VP1, VP2 and VP3, which interact together to form the viral capsid.

Recombinant AAV (rAAV) vectors may comprise a nucleic acid vector, which may comprise at a minimum (a) one or more heterologous nucleic acid regions comprising a sequence encoding a protein or polypeptide of interest and (b) one or more regions comprising inverted terminal repeat (ITR) sequences (e.g., wild-type ITR sequences or engineered ITR sequences) flanking the one or more heterologous nucleic acid regions. This nucleic acid vector may be encapsidated by a viral capsid. In some embodiments, the nucleic acid vector is circular. In some embodiments, the nucleic acid vector is single-stranded. In some embodiments, the nucleic acid vector is double-stranded. In some embodiments, a double-stranded nucleic acid vector may be, for example, a self-complimentary vector that contains a region of the nucleic acid vector that is complementary to another region of the nucleic acid vector, initiating the formation of the double-strandedness of the nucleic acid vector.

Accordingly, in some embodiments, a rAAV vector comprises a viral capsid and a nucleic acid vector as described herein, which is encapsidated by the viral capsid. In some embodiments, the nucleic acid vector comprises (1) one or more heterologous nucleic acid regions comprising a sequence encoding a protein or polypeptide of interest, (2) one or more nucleic acid regions comprising a sequence that facilitates expression of the heterologous nucleic acid region (e.g., a promoter and/or enhancer), and (3) one or more nucleic acid regions comprising a sequence that facilitate integration of the heterologous nucleic acid region (optionally with the one or more nucleic acid regions comprising a sequence that facilitates expression) into the genome of the subject. In some embodiments, viral sequences that facilitate integration comprise Inverted Terminal Repeat (ITR) sequences. In some embodiments, the nucleic acid vector comprises one or more heterologous nucleic acid regions comprising a sequence encoding a protein or polypeptide of interest operably linked to a promoter, wherein the one or more heterologous nucleic acid regions are flanked on each side with an ITR sequence. The ITR sequences can be derived from any AAV serotype (e.g., 1, 2, 3, 4, 5, 6, 7, 8, 9, 10) or can be derived from more than one serotype. In some embodiments, the ITR sequences are derived from AAV2. ITR sequences and plasmids containing ITR sequences are known in the art and commercially available (see, e.g., products and services available from Vector Biolabs, Philadelphia, PA; Cellbiolabs, San Diego, CA; Agilent Technologies, Santa Clara, Ca; and Addgene, Cambridge, MA; and Gene delivery to skeletal muscle results in sustained expression and systemic delivery of a therapeutic protein. Kessler PD, Podsakoff GM, Chen X, McQuiston SA, Colosi PC, Matelis LA, Kurtzman GJ, Byrne BJ. Proc Natl Acad Sci U S A. 1996 Nov 26;93(24):14082-7; and Curtis A. Machida. Methods in Molecular Medicine™. Viral Vectors for Gene TherapyMethods and Protocols. 10.1385/1-59259-304-6:201 © Humana Press Inc. 2003. Chapter 10. Targeted Integration by Adeno-Associated Virus. Matthew D. Weitzman, Samuel M. YoungJr., Toni Cathomen and Richard Jude Samulski; U.S. Pat. Nos. 5,139,941 and 5,962,313, all of which are incorporated herein by reference).

In some embodiments, the nucleic acid vector comprises a pTR-UF-11 plasmid backbone, which is a plasmid that contains AAV2 ITRs. This plasmid is commercially available from the American Type Culture Collection (ATCC MBA-331).

In some embodiments, the nucleic acid vector comprises one or more regions comprising a sequence that facilitates expression of the heterologous nucleic acid, e.g., expression control sequences operatively linked to the heterologous nucleic acid. Numerous such sequences are known in the art. Non-limiting examples of expression control sequences include promoters, insulators, silencers, response elements, introns, enhancers, initiation sites, termination signals, and poly(A) tails. Any combination of such control sequences is completed herein (e.g., a promoter and an enhancer).

To achieve appropriate expression levels of the protein or polypeptide of interest, any of a number of promoters suitable for use in the selected host cell may be employed. The promoter may be, for example, a constitutive promoter, tissue-specific promoter, inducible promoter, or a synthetic promoter. For example, constitutive promoters of different strengths can be used. A nucleic acid vector described herein may include one or more constitutive promoters, such as viral promoters or promoters from mammalian genes that are generally active in promoting transcription. Non-limiting examples of constitutive viral promoters include the Herpes Simplex virus (HSV), thymidine kinase (TK), Rous Sarcoma Virus (RSV), Simian Virus 40 (SV40), Mouse Mammary Tumor Virus (MMTV), Ad E1A and cytomegalovirus (CMV) promoters. Non-limiting examples of constitutive mammalian promoters include various housekeeping gene promoters, as exemplified by the β-actin promoter.

Inducible promoters and/or regulatory elements may also be contemplated for achieving appropriate expression levels of the protein or polypeptide of interest. Non-limiting examples of suitable inducible promoters include those from genes such as cytochrome P450 genes, heat shock protein genes, metallothionein genes, and hormone-inducible genes, such as the estrogen gene promoter. Another example of an inducible promoter is the tetVP16 promoter that is responsive to tetracycline.

Tissue-specific promoters and/or regulatory elements are also contemplated herein. Non-limiting examples of such promoters that may be used include (1) desmin, creatine kinase, myogenin, alpha myosin heavy chain, human brain and natriuretic peptide, specific for muscle cells, and (2) albumin, alpha-1-antitrypsin, hepatitis B virus core protein promoters, specific for liver cells.

Synthetic promoters are also contemplated herein. A synthetic promoter may comprise, for example, regions of known promoters, regulatory elements, transcription factor binding sites, enhancer elements, repressor elements, and the like.

In some embodiments, a nucleic acid vector described herein may also contain marker or reporter genes, e.g., LacZ or a fluroscent protein.

In some embodiments, the nucleic acid vector comprises one or more heterologous nucleic acid regions comprising a sequence encoding a protein or polypeptide of interest. The protein or polypeptide of interest may be, e.g., a polypeptide or protein of interest provided in Table 1. The sequences of the polypeptide or protein of interest may be obtained, e.g., using the non-limiting National Center for Biotechnology Information (NCBI) Protein IDs or SEQ ID NOs from patent applications provided in Table 1.

**Table 1. Non-limiting examples of proteins or polypeptides of interest and associated diseases**

| **Protein or Polypeptide** | **Non-limiting Exemplary diseases** | **Non-limiting NCBI Protein IDs or Patent SEQ ID NOs** |
|---|---|---|
| acid alpha-glucosidase (GAA) | Pompe | NP_000143.2, NP_001073271.1, NP 001073272.1 |
| Methyl CpG binding protein 2 (MECP2) | Rett syndrome | NP _001104262.1, NP_ 004983.1 |
| Aromatic L-amino acid decarboxylase (AADC) | Parkinson's disease | NP_000781.1, NP_001076440.1, NP_001229815.1, NP 001229816.1, |
| | | NP_001229817.1, NP_001229818.1, NP 001229819.1 |
| Glial cell-derived neurotrophic factor (GDNF) | Parkinson's disease | NP_000505.1, NP _001177397.1, NP _001177398.1, NP _001265027.1, NP_ 954701.1 |
| Cystic fibrosis transmembrane conductance regulator (CFTR) | Cystic fibrosis | NP_000483.3 |
| Tumor necrosis factor receptor fused to an antibody Fc (TNFR:Fc) | Arthritis, Rheumatoid arthritis | SEQ ID NO. 1 of WO2013025079 |
| HIV-1 gag-proΔ*rt* (tgAAC09) | HIV infection | SEQ ID NOs. 1-5 of WO2006073496 |
| Sarcoglycan alpha, beta, gamma, delta, epsilon, or zeta (SGCA, SGCB, SGCG, SGCD, SGCE, or SGCZ) | Muscular dystrophy | SGCA NP_000014.1, NP_001129169.1 |
| | | SGCB NP_000223.1 |
| | | SGCG NP_000222.1 |
| | | SGCD NP_000328.2, NP_001121681.1, NP_758447.1 |
| | | SGCE NP_001092870.1, NP_001092871.1, NP_003910.1 |
| | | SGCZ NP 631906.2 |
| Alpha-1-antitrypsin (AAT) | Hereditary emphysema or Alpha-1 -antitrypsin deficiency | NP _000286.3, NP _001002235.1, NP _001002236.1, NP _001121172.1, NP _001121173.1, NP _001121174.1, NP _001121175.1, NP 001121176.1, |
| | | NP_001121177.1, NP_001121178.1, NP 001121179.1 |
| Glutamate decarboxylase 1(GAD1) | Parkinson's disease | NP _000808.2, NP 038473.2 |
| Glutamate decarboxylase 2 (GAD2) | Parkinson's disease | NP _000809.1, NP 001127838.1 |
| Aspartoacylase (ASPA) | Canavan's disease | NP_000040.1, NP 001121557.1 |
| Nerve growth factor (NGF) | Alzheimer's disease | NP_ 002497.2 |
| Granulocyte-macrophage colonystimulating factory (GM-CSF) | Prostate cancer | NP_000749.2 |
| Cluster of Differentiation 86 (CD86 or B7-2) | Malignant melanoma | NP _001193853.1, NP _001193854.1, NP _008820.3, NP _787058.4, NP _795711.1 |
| Interleukin 12 (IL-12) | Malignant melanoma | NP_000873.2, NP_002178.2 |
| neuropeptide Y (NPY) | Parkinson's disease, epilepsy | NP _000896.1 |
| ATPase, Ca++ transporting, cardiac muscle, slow twitch 2 (SERCA2) | Chronic heart failure | NP _001672.1, NP _733765.1 |
| Dystrophin or Minidystrophin | Muscular dystrophy | NP _000100.2, NP _003997.1, NP _004000.1, NP _004001.1, NP _004002.2, NP _004003.1, NP _004004.1, NP_004005.1, NP _004006.1, NP _004007.1, NP _004008.1, NP _004009.1, NP _004010.1, NP _004011.2, NP _004012.1, NP _004013.1, NP _004014.1 |
| Ceroid lipofuscinosis neuronal 2 (CLN2) | Late infantile neuronal ceroidlipofuscinosis or Batten's disease | NP_000382.3 |
| Neurturin (NRTN) | Parkinson's disease | NP _004549.1 |
| N-acetylglucosaminidase, alpha (NAGLU) | Sanfilippo syndrome (MPSIIIB) | NP_000254.2 |
| Iduronidase, alpha-1 (IDUA) | MPSI-Hurler | NP _000194.2 |
| Iduronate 2-sulfatase (IDS) | MPSII-Hunter | NP_000193.1, NP_001160022.1, NP_006114.1 |
| Glucuronidase, beta (GUSB) | MPSVII-Sly | NP _000172.2, NP _001271219.1 |
| Hexosaminidase A, α polypeptide (HEXA) | Tay-Sachs | NP_000511.2 |
| Retinal pigment epithelium-specific protein 65kDa (RPE65) | Leber congenital amaurosis | NP_000320.1 |
| Factor IX (FIX) | Hemophilia B | NP _000124.1 |
| Adenine nucleotide translocator (ANT-1) | progressive external ophthalmoplegia | NP_001142.2 |
| ApaLI | mitochondrial heteroplasmy, myoclonic epilepsy with ragged red fibers (MERRF) or mitochondrial encephalomy opathy, lactic acidosis, and stroke-like episodes (MELAS) | YP_007161330.1 |
| NADH ubiquinone oxidoreductase subunit 4 (ND4) | Leber hereditary optic | YP_003024035.1 |
| very long-acyl-CoA dehydrogenase (VLCAD) | very long-chain acyl-CoA dehydrogenase (VLCAD) deficiency | NP _000009.1, NP _001029031.1, NP _001257376.1, NP _001257377.1 |
| short-chain acyl-CoA dehydrogenase (SCAD) | short-chain acyl-CoA dehydrogenase (SCAD) deficiency | NP_000008.1 |
| medium-chain acyl-CoA dehydrogenase (MCAD) | medium-chain acyl-CoA dehydrogenase (MCAD) deficiency | NP_000007.1, NP_001120800.1, NP_001272971.1, NP_001272972.1, NP_001272973.1 |
| Myotubularin 1 (MTM1) | X-linked myotubular myopathy | NP _000243.1 |
| Myophosphorylase (PYGM) | McArdle disease (glycogen storage disease type V, myophosphorylase deficiency) | NP_001158188.1, NP_005600.1 |
| Lipoprotein lipase (LPL) | LPL deficiency | NP _000228.1 |
| sFLT01 (VEGF/PlGF (placental growth factor) binding domain of | Age-related macular degeneration | SEQ ID NO: 2, 8, 21, 23, or 25 of |
| human VEGFR1/Flt-1 (hVEGFR1) fused to the Fc portion of human IgG(1) through a polyglycine linker) | | WO2009105669 |
| Glucocerebrosidase (GC) | Gaucher disease | NP _000148.2, NP _001005741.1, NP _001005742.1, NP _001165282.1, NP 001165283.1 |
| UDP glucuronosyltransferase 1 family, polypeptide A1 (UGT1A1) | Crigler-Najjar syndrome | NP_000454.1 |
| Glucose 6-phosphatase (G6Pase) | GSD-Ia | NP _000142.2, NP _001257326.1 |
| Ornithine carbamoyltransferase (OTC) | OTC deficiency | NP _000522.3 |
| Cystathionine-beta-synthase (CBS) | Homocystinuria | NP_000062.1, NP_001171479.1, NP_001171480.1 |
| Factor VIII (F8) | Haemophilia A | NP _000123.1, NP _063916.1 |
| Hemochromatosis (HFE) | Hemochromatosis | NP_000401.1, NP_620572.1, NP_620573.1, NP_620575.1, NP_620576.1, NP_620577.1, NP_620578.1, NP_620579.1, NP_620580.1 |
| Low density lipoprotein receptor (LDLR) | Phenylketonuria (PKU) | NP_000518.1, NP_001182727.1, NP_001182728.1, NP_001182729.1, NP_001182732.1 |
| Galactosidase, alpha (AGA) | Fabry disease | NP _000160.1 |
| Phenylalanine hydroxylase (PAH) | Hypercholesterolaemia or Phenylketonuria (PKU) | NP_000268.1 |
| Propionyl CoA carboxylase, alpha polypeptide (PCCA) | Propionic acidaemias | NP_000273.2, NP_001121164.1, NP 001171475.1 |

The polypeptides and proteins provided in Table 1 are known in the art (see, e.g., Adeno-Associated Virus Vectors in Clinical Trials. Barrie J. Carter. Human Gene Therapy. May 2005, 16(5): 541-550. doi:10.1089/hum.2005.16.541. Published in Volume: 16 Issue 5: May 25, 2005; Neuropharmacology. 2013 Jun;69:82-8. doi: 10.1016/j.neuropharm.2012.03.004. Epub 2012 Mar 17.; Adeno-associated virus (AAV) gene therapy for neurological disease. Weinberg MS1, Samulski RJ, McCown TJ.Gene therapy for lysosomal storage disorders. Yew NS, Cheng SH. Pediatr Endocrinol Rev. 2013 Nov;11 Suppl 1:99-109; Directed evolution of novel adeno-associated viruses for therapeutic gene delivery. Bartel MA, Weinstein JR, Schaffer DV.Gene Ther. 2012 Jun;19(6):694-700. doi: 10.1038/gt.2012.20. Epub 2012 Mar 8; Therapeutic in vivo gene transfer for genetic disease using AAV: progress and challenges. Mingozzi F, High KA. Nat Rev Genet. 2011 May;12(5):341-55. doi: 10.1038/nrg2988). In some embodiments, the polypeptide or protein of interest is a human protein or polypeptide.

In some embodiments, the protein or polypeptide of interest is acid alpha-glucosidase. In some embodiments, the protein or polypeptide of interest is not rituximab.

The rAAV vector may be of any AAV serotype, including any derivative (including non-naturally occurring variants) or pseudotype (e.g., 1, 2, 3, 4, 5, 6, 7, 8, 9, 10, 2/1, 2/5, 2/8, or 2/9). Non-limiting examples of derivatives and pseudotypes include rAAV2/1, rAAV2/5, rAAV2/8, rAAV2/9, AAV2-AAV3 hybrid, AAVrh.10,AAVhu.14, AAV3a/3b, AAVrh32.33, AAV-HSC15, AAV-HSC17, AAVhu.37, AAVrh.8, CHt-P6, AAV2.5, AAV6.2, AAV2i8, AAV-HSC15/17, AAVM41, AAV9.45, AAV6(Y445F/Y731F), AAV2.5T, AAV-HAE1/2, AAV clone 32/83, AAVShH10, AAV2 (Y->F), AAV8 (Y733F), AAV2.15, AAV2.4, AAVM41, and AAVr3.45. Such AAV serotypes and derivatives/pseudotypes, and methods of producing such derivatives/pseudotypes are known in the art (see, e.g., Mol Ther. 2012 Apr;20(4):699-708. doi: 10.1038/mt.2011.287. Epub 2012 Jan 24. The AAV vector toolkit: poised at the clinical crossroads. Asokan A1, Schaffer DV, Samulski RJ.). In some embodiments, the rAAV vector is a pseudotyped rAAV vector. In some embodiments, the pseudotyped rAAV vector is a rAAV2/x vector, which comprises (a) a nucleic acid vector comprising AAV2 ITRs and (b) a capsid comprised of capsid proteins derived from AAVx (e.g., AAV1, AAV3, AAV4, AAV5, AAV6, AAV7, AAV8, AAV9, or AAV10). Exemplary rAAV pseudotyped vectors include, but are not limited to rAAV2/1, rAAV2/5, rAAV2/8, and rAAV2/9 vectors. Methods for producing and using pseudotyped rAAV vectors are known in the art (see, e.g., Duan et al., J. Virol., 75:7662-7671, 2001; Halbert et al., J. Virol., 74:1524-1532, 2000; Zolotukhin et al., Methods, 28:158-167, 2002; and Auricchio et al., Hum. Molec. Genet., 10:3075-3081, 2001).

In some embodiments, a prior and a subsequent rAAV vector (e.g., a second, third, fourth, fifth, sixth, seventh, eighth, ninth, tenth, or more) are contemplated for use in a method or kit herein.

In some embodiments, the prior and subsequent rAAV vectors are the same serotype (e.g., both AAV1, both AAV2, both AAV3, both AAV4, both AAV5, both AAV6, both AAV7, both AAV8, both AAV9, both AAV10, both AAV2/1, both rAAV2/5, both rAAV2/8, or both rAAV2/9). Accordingly, in some embodiments, a method provided herein comprises administering a B cell depletion treatment and a subsequent rAAV vector as described herein to the human or non-human primate subject that has previously been administered a prior rAAV vector as described herein, wherein the subsequent rAAV vector and the prior rAAV vector are the same serotype. In some embodiments, the method comprises administering an anti-CD20 antibody as described herein (e.g., rituximab) and a subsequent rAAV vector as described herein to a human or non-human primate subject that has previously been administered a prior rAAV vector as described herein, wherein the subsequent rAAV vector and the prior rAAV vector are the same serotype.

In some embodiments, the prior and subsequent rAAV vectors are different serotypes (e.g., one is AAV1 and the other is AAV2, one is AAV2 and the other is AAV8, one is AAV8 and the other is AAV9, one is AAV2/1 and the other is AAV2/8, one is AAV2/1 and the other is AAV2/9, or one is AAV2/8 and the other is AAV2/9). In some embodiments, the prior rAAV vector comprises a nucleic acid vector that encode a first protein or polypeptide of interest and the subsequent rAAV vector comprises a nucleic acid vector that encodes a second protein or polypeptide of interest. In some embodiments, the first and second protein or polypeptide of interest are the same protein or polypeptide of interest (e.g., both the first and second protein or polypeptide of interest are GAA). In some embodiments, the first and second protein or polypeptide of interest are different proteins or polypeptides of interest (e.g., the first protein or polypeptide of interest is GAA and the second protein or polypeptide of interest is a protein or polypeptide other than GAA, or vice versa).

Methods of producing rAAV vectors and nucleic acid vectors are known in the art and commercially available (see, e.g., Zolotukhin et al. Production and purification of serotype 1, 2, and 5 recombinant adeno-associated viral vectors. Methods 28 (2002) 158-167; and U.S. Patent Publication Numbers US20070015238 and US20120322861, which are incorporated herein by reference; and plasmids and kits available from ATCC and Cell Biolabs, Inc.). For example, the nucleic acid vector may be combined with one or more helper plasmids that encode *rep* and *cap* ORFS and transfected into a producer cell line such that the rAAV vector can be packaged and subsequently purified. Helper plasmids, and methods of making such plasmids, are known in the art and commercially available (see, e.g., pDM, pDG, pDP1rs, pDP2rs, pDP3rs, pDP4rs, pDP5rs, pDP6rs, pDG(R484E/R585E), and pDP8.ape plasmids from PlasmidFactory, Bielefeld, Germany; other products and services available from Vector Biolabs, Philadelphia, PA; Cellbiolabs, San Diego, CA; Agilent Technologies, Santa Clara, Ca; and Addgene, Cambridge, MA; Grimm et al. (1998), Novel Tools for Production and Purification of Recombinant Adenoassociated Virus Vectors, Human Gene Therapy, Vol. 9, 2745-2760; Kern, A. et al. (2003), Identification of a Heparin-Binding Motif on Adeno-Associated Virus Type 2 Capsids, Journal of Virology, Vol. 77, 11072-11081.; Grimm et al. (2003), Helper Virus-Free, Optically Controllable, and Two-Plasmid-Based Production of Adeno-associated Virus Vectors of Serotypes 1 to 6, Molecular Therapy,Vol. 7, 839-850; Kronenberg et al. (2005), A Conformational Change in the Adeno-Associated Virus Type 2 Capsid Leads to the Exposure of Hidden VP1 N Termini, Journal of Virology, Vol. 79, 5296-5303; and Moullier, P. and Snyder, R.O. (2008), International efforts for recombinant adenoassociated viral vector reference standards, Molecular Therapy, Vol. 16, 1185-1188).

An exemplary, non-limiting, rAAV vector production method is described next. A helper plasmid is produced or obtained, which comprises *rep* and *cap* ORFs and the adenoviral VA, E2A (DBP), and E4 genes under the transcriptional control of their native promoters. 293 cells (available from ATCC) are transfected with the helper plasmid and a nucleic acid vector described herein (e.g., comprising one or more heterologous sequences that encode a protein or polypeptide of interest and ITRs flanking the heterologous sequences). The 293 cells are then incubated for at least 24 hours to allow for rAAV vector production. The 293 cells are then washed, centrifuged, and lysed using freeze/thaw cycles. Benzonase is added to further digest the lysed cells. The rAAV vectors present in the lysate are then purified using iodixanol gradient centrifugation and heparin affinity resin or iodixanol gradient centrifugation and anion-exchange chromatography. The rAAV vectors may optionally be concentrated, desalted, and/or buffer exchanged using commercially available concentrators (see, e.g., products available from EMD Millipore). The rAAV vector is then stored in lactated Ringer's or PBS.

It is to be understood that other viral vectors suitable for gene therapy are also contemplated for use in a method or kit described herein, e.g., a lentivirus vector for delivery of one or more heterologous nucleic acid sequences encoding a protein or polypeptide of interest into various tissues, organs, and/or cells. In some embodiments, a lentivirus vector comprises a nucleic acid vector, which may comprise at a minimum (a) one or more heterologous nucleic acid regions comprising a sequence encoding a protein or polypeptide of interest and (b) one or more long terminal repeat (LTR) sequences (e.g., wild-type LTR sequences or engineered LTR sequences) flanking the one or more heterologous nucleic acid regions. In some embodiments, the nucleic acid vector may comprise any other nucleic acid region described herein (e.g., a promoter, enhancer, etc.). Lentiviral vectors, and methods of making lentiviral vectors, are known in the art and commercially available (see, e.g., products available from Addgene, Cambridge, MA).

Other methods for obtaining, producing, making and using rAAV vectors, e.g., for treatment, are known in the art and discussed herein (see, e.g., Kessler et al. (1996). Gene delivery to skeletal muscle results in sustained expression and systemic delivery of a therapeutic protein. Proceedings of the National Academy of Sciences of the United States of America 93: 14082-14087; Pauly et al. (1998). Complete correction of acid alpha-glucosidase deficiency in Pompe disease fibroblasts in vitro, and lysosomally targeted expression in neonatal rat cardiac and skeletal muscle. Gene therapy 5: 473-480; Song et al. (1998). Sustained secretion of human alpha-1-antitrypsin from murine muscle transduced with adeno-associated virus vectors. Proceedings of the National Academy of Sciences of the United States of America 95: 14384-14388; Conway et al. (1999). High-titer recombinant adeno-associated virus production utilizing a recombinant herpes simplex virus type I vector expressing AAV-2 Rep and Cap. Gene therapy 6: 986-993; Xu et al. (2001). CMV-beta-actin promoter directs higher expression from an adeno-associated viral vector in the liver than the cytomegalovirus or elongation factor 1 alpha promoter and results in therapeutic levels of human factor X in mice. Human gene therapy 12: 563-573; Asfour et al. (2002). Uniform long-term gene expression using adeno-associated virus (AAV) by ex vivo recirculation in rat-cardiac isografts. The Thoracic and cardiovascular surgeon 50: 347-350; Fraites et al. (2002). Correction of the enzymatic and functional deficits in a model of Pompe disease using adeno-associated virus vectors. Molecular therapy : the journal of the American Society of Gene Therapy 5: 571-578; Mah et al. (2002). Virus-based gene delivery systems. Clinical pharmacokinetics 41: 901-911; Mah et al. (2002). Improved method of recombinant AAV2 delivery for systemic targeted gene therapy. Molecular therapy : the journal of the American Society of Gene Therapy 6: 106-112; Sun et al. (2002). Sustained hepatic and renal glucose-6-phosphatase expression corrects glycogen storage disease type Ia in mice. Human molecular genetics 11: 2155-2164; Zolotukhin et al. (2002). Production and purification of serotype 1, 2, and 5 recombinant adeno-associated viral vectors. Methods 28: 158-167; Mah et al. (2003). Dual vectors expressing murine factor VIII result in sustained correction of hemophilia A mice. Human gene therapy 14: 143-152; Flotte et al. (2004). Phase I trial of intramuscular injection of a recombinant adeno-associated virus alpha 1-antitrypsin (rAAV2-CB-hAAT) gene vector to AAT-deficient adults. Human gene therapy 15: 93-128; Mah et al. (2004). A new method for recombinant adeno-associated virus vector delivery to murine diaphragm. Molecular therapy : the journal of the American Society of Gene Therapy 9: 458-463; Rucker et al. (2004). Rescue of enzyme deficiency in embryonic diaphragm in a mouse model of metabolic myopathy: Pompe disease. Development 131: 3007-3019; Cresawn et al. (2005). Impact of humoral immune response on distribution and efficacy of recombinant adeno-associated virus-derived acid alpha-glucosidase in a model of glycogen storage disease type II. Human gene therapy 16: 68-80; Kohlbrenner et al. (2005). Successful production of pseudotyped rAAV vectors using a modified baculovirus expression system. Molecular therapy : the journal of the American Society of Gene Therapy 12: 1217-1225; Mah et al. (2005). Sustained correction of glycogen storage disease type II using adeno-associated virus serotype 1 vectors. Gene therapy 12: 1405-1409; Walker et al. (2005). Expression of erythropoietin in cats treated with a recombinant adeno-associated viral vector. American journal of veterinary research 66: 450-456; Brantly et al. (2006). Phase I trial of intramuscular injection of a recombinant adeno-associated virus serotype 2 alphal-antitrypsin (AAT) vector in AAT-deficient adults. Human gene therapy 17: 1177-1186; Ghosh et al. (2006). Long-term correction of murine glycogen storage disease type Ia by recombinant adeno-associated virus-1-mediated gene transfer. Gene therapy 13: 321-329; Kishnani et al. (2006). Pompe disease diagnosis and management guideline. Genetics in medicine : official journal of the American College of Medical Genetics 8: 267-288; Pacak et al. (2006). Recombinant adeno-associated virus serotype 9 leads to preferential cardiac transduction in vivo. Circ Res 99: e3-9; Flotteet et al. (2007). Preclinical characterization of a recombinant adeno-associated virus type 1-pseudotyped vector demonstrates dose-dependent injection site inflammation and dissemination of vector genomes to distant sites. Human gene therapy 18: 245-256; Mah et al. (2007). Physiological correction of Pompe disease by systemic delivery of adeno-associated virus serotype 1 vectors. Molecular therapy : the journal of the American Society of Gene Therapy 15: 501-507; Pacak et al. (2007). Long-term skeletal muscle protection after gene transfer in a mouse model of LGMD-2D. Molecular therapy : the journal of the American Society of Gene Therapy 15: 1775-1781; Cideciyan et al. (2008). Human gene therapy for RPE65 isomerase deficiency activates the retinoid cycle of vision but with slow rod kinetics. Proceedings of the National Academy of Sciences of the United States of America 105: 15112-15117; Flotte et al. (2008). Apparently nonspecific enzyme elevations after portal vein delivery of recombinant adeno-associated virus serotype 2 vector in hepatitis C virus-infected chimpanzees. Human gene therapy 19: 681-689; Hauswirth et al. (2008). Treatment of leber congenital amaurosis due to RPE65 mutations by ocular subretinal injection of adeno-associated virus gene vector: short-term results of a phase I trial. Human gene therapy 19: 979-990; Pacak et al. (2008). Relative persistence of AAV serotype 1 vector genomes in dystrophic muscle. Genetic vaccines and therapy 6: 14; Pacak et al. (2008). Tissue specific promoters improve specificity of AAV9 mediated transgene expression following intra-vascular gene delivery in neonatal mice. Genetic vaccines and therapy 6: 13; Polyak et al. (2008). Gene delivery to intestinal epithelial cells in vitro and in vivo with recombinant adeno-associated virus types 1, 2 and 5. Digestive diseases and sciences 53: 1261-1270; Brantly et al. (2009). Sustained transgene expression despite T lymphocyte responses in a clinical trial of rAAV1-AAT gene therapy. Proceedings of the National Academy of Sciences of the United States of America 106: 16363-16368; Byrne, BJ (2009). Innovative vector design: cross-packaged, self-complementary and now trans-splicing AAV vectors. Human gene therapy 20: 1224-1225; Cideciyan et al. (2009). Human RPE65 gene therapy for Leber congenital amaurosis: persistence of early visual improvements and safety at 1 year. Human gene therapy 20: 999-1004; Cideciyan et al. (2009). Vision 1 year after gene therapy for Leber's congenital amaurosis. The New England journal of medicine 361: 725-727; Clement et al. (2009). Large-scale adeno-associated viral vector production using a herpesvirus-based system enables manufacturing for clinical studies. Human gene therapy 20: 796-806; Mendell et al. (2009). Limb-girdle muscular dystrophy type 2D gene therapy restores alpha-sarcoglycan and associated proteins. Annals of neurology 66: 290-297; Lock et al. (2010). Characterization of a recombinant adeno-associated virus type 2 Reference Standard Material. Human gene therapy 21: 1273-1285; Mah et al. (2010). Gel-mediated delivery of AAV1 vectors corrects ventilatory function in Pompe mice with established disease. Molecular therapy : the journal of the American Society of Gene Therapy 18: 502-510; Mendell et al. (2010). Sustained alpha-sarcoglycan gene expression after gene transfer in limb-girdle muscular dystrophy, type 2D. Annals of neurology 68: 629-638; Weinstein et al. (2010). Adeno-associated virus-mediated correction of a canine model of glycogen storage disease type Ia. Human gene therapy 21: 903-910; Byrne et al. (2011). Pompe disease gene therapy. Human molecular genetics 20: R61-68; Mandel et al. (2011). AAV6-mediated gene silencing fALS short. Molecular therapy : the journal of the American Society of Gene Therapy 19: 231-233; Pacak et al. (2011). AAV vectors for cardiac gene transfer: experimental tools and clinical opportunities. Molecular therapy : the journal of the American Society of Gene Therapy 19: 1582-1590; Byrne et al. (2012). Gene therapy approaches for lysosomal storage disease: next-generation treatment. Human gene therapy 23: 808-815; ElMallah et al. (2012). Retrograde gene delivery to hypoglossal motoneurons using adeno-associated virus serotype 9. Human gene therapy methods 23: 148-156; Jacobson et al. (2012). Gene therapy for leber congenital amaurosis caused by RPE65 mutations: safety and efficacy in 15 children and adults followed up to 3 years. Archives of ophthalmology 130: 9-24; Lee et al. (2012). An acidic oligopeptide displayed on AAV2 improves axial muscle tropism after systemic delivery. Genetic vaccines and therapy 10: 3; Qiu et al. (2012). Spinal Delivery of AAV Vector Restores Enzyme Activity and Increases Ventilation in Pompe Mice. Molecular therapy: the journal of the American Society of Gene Therapy 20: 21-27; Stedman et al. (2012). Signs of progress in gene therapy for muscular dystrophy also warrant caution. Molecular therapy: the journal of the American Society of Gene Therapy 20: 249-251; Conlon et al. (2013). Preclinical toxicology and biodistribution studies of recombinant adeno-associated virus 1 human acid alpha-glucosidase. Human gene therapy Clinical development 24: 127-133; Elmallah et al. (2013). Sustained Correction of Motoneuron Histopathology Following Intramuscular Delivery of AAV in Pompe Mice. Molecular therapy : the journal of the American Society of Gene Therapy; Falk et al. (2013). Intrapleural administration of AAV9 improves neural and cardiorespiratory function in Pompe disease. Molecular therapy : the journal of the American Society of Gene Therapy 21: 1661-1667; Lee et al. (2013). Treatment of congenital neurotransmitter deficiencies by intracerebral ventricular injection of an AAV9 vector. Human gene therapy; Mah et al. (2013). Adeno-associated virus-mediated gene therapy for metabolic myopathy. Human gene therapy 24: 928-936; Smith et al. (2013). Phase I/II trial of adeno-associated virus-mediated alpha-glucosidase gene therapy to the diaphragm for chronic respiratory failure in Pompe disease: initial safety and ventilatory outcomes. Human gene therapy 24: 630-640, all of which are incorporated by reference with respect to the disclosure related to obtaining, producing, making, and using rAAV vectors).

### Subjects

Aspects of the disclosure relate to methods and kits for use with human and non-human primate subjects. Non-limiting examples of non-human primate subjects include macaques (e.g., cynomolgus or rhesus macaques), marmosets, tamarins, spider monkeys, owl monkeys, vervet monkeys, squirrel monkeys, baboons, gorillas, chimpanzees, and orangutans. In some embodiments, the subject is a human subject. In some embodiments, the subject is a juvenile human subject, such a human subject under the age of 18 years. In some embodiments, the subject is a human subject under the age of 5, 4, 3, 2 or 1 year. In some embodiments, the subject is a subject that has not been previously exposed to wild-type AAV or administered a rAAV vector. In some embodiments, the subject is a subject that has not been previously administered a rAAV vector. In some embodiments, the subject is a subject that has been previously administered a rAAV vector, e.g., a rAAV vector described herein. A subject that has been exposed or administered an AAV or rAAV can be identified using methods known in the art, e.g., by PCR detection of viral DNA or by measuring antibody titer to AAV or rAAV, either the capsid or the transgene. In some embodiments, the subject is a subject that has not been administered an enzyme replacement therapy (e.g., by administration of the enzyme protein). A subject that has been administered an enzyme replacement therapy can be identified using methods known in the art, e.g., by measuring antibody titer to the enzyme. However, in some embodiments the subject has previously been treated with an enzyme replacement therapy.

In some embodiments, the subject has or is suspected of having a disease that may be treated with gene therapy. Exemplary diseases include, but are not limited to, cystic fibrosis, hemophilia B, San Filippo syndrome, lipoprotein lipase deficiency, alpha-1 antitrypsin deficiency, arthritis, hereditary emphysema, Leber's congenital amaurosis, age-related macular degeneration, muscular dystrophy (duchenne, LGMD2d and 2c), Parkinson's disease, Canavan's disease, Batten's disease, Alzheimer's disease, metachromatic leukodystrophy, alpha-1 antitrypsin deficiency, lipoprotein lipase deficiency, heart failure, rheumatoid arthritis, amyotrophic lateral sclerosis (ALS), spinal muscular atrophy (SMA), ornithine transcarbamylase deficiency, epilepsy, Rett syndrome,lysosomal storage disorders of skeletal muscle or CNS or Pompe disease, or another disease listed in Table 1. These diseases, associated symptoms and signs of the diseases, and methods of diagnosis of the diseases are known in the art and available to the skilled practitioner. Treatment methods involving rAAV vectors are also known in the art (see, e.g., Adeno-Associated Virus Vectors in Clinical Trials. Barrie J. Carter. Human Gene Therapy. May 2005, 16(5): 541-550. doi:10.1089/hum.2005.16.541. Published in Volume: 16 Issue 5: May 25, 2005; Neuropharmacology. 2013 Jun;69:82-8. doi: 10.1016/j.neuropharm.2012.03.004. Epub 2012 Mar 17.; Adeno-associated virus (AAV) gene therapy for neurological disease. Weinberg MS1, Samulski RJ, McCown TJ.Gene therapy for lysosomal storage disorders. Yew NS, Cheng SH. Pediatr Endocrinol Rev. 2013 Nov;11 Suppl 1:99-109; Directed evolution of novel adeno-associated viruses for therapeutic gene delivery. Bartel MA, Weinstein JR, Schaffer DV.Gene Ther. 2012 Jun;19(6):694-700. doi: 10.1038/gt.2012.20. Epub 2012 Mar 8; Therapeutic in vivo gene transfer for genetic disease using AAV: progress and challenges. Mingozzi F, High KA. Nat Rev Genet. 2011 May;12(5):341-55. doi: 10.1038/nrg2988).

In some embodiments, the subject has Pompe disease. Pompe disease is a lysosomal storage disease caused by an accumulation of glycogen in the lysosome due to deficiency of the lysosomal acid alpha-glucosidase enzyme. The build-up of glycogen causes progressive myopathy throughout the body and affects various tissues, particularly in the heart, skeletal muscles, liver and nervous system.

### Anti-CD20 antibodies

Aspects of the disclosure relate to anti-CD20 antibodies for use in a method or kit described herein. An anti-CD20 antibody is an antibody that specifically binds CD20. An antibody that "specifically binds" (used interchangeably herein) to a target (e.g., CD20) is a term well understood in the art, and methods to determine such specific or preferential binding are also well known in the art. A molecule is said to exhibit "specific binding" if it binds with to the antigen with greater affinity, avidity, more readily, and/or with greater duration than it binds to other substances. For example, an antibody that specifically binds CD20, or an epitope of CD20, is an antibody that binds CD20, or an epitope of CD20, with greater affinity, avidity, more readily, and/or with greater duration than it binds to other non-CD20 antigens or epitopes. "Specific binding" does not necessarily require (although it can include) exclusive binding. Generally, but not necessarily, reference to binding means preferential binding.

As used herein, an antibody encompasses not only intact polyclonal or monoclonal antibodies, but also antigen-binding fragments thereof (such as Fab, Fab', F(ab')2, Fv), single chain (ScFv) and domain antibodies (including, for example, shark and camelid antibodies), and fusion proteins comprising an antibody, and any other modified configuration of the immunoglobulin molecule that comprises an antigen recognition site. In some embodiments, the antibody can be a human antibody, a humanized antibody, or a chimeric antibody. In some embodiments, the antibody is a monoclonal antibody. In some embodiments, the antibody comprises a constant region. In some embodiments, the antibody is of the human lgG1, lgG2 or lgG2Aa, lgG3, or lgG4 subclass. In some embodiments, the antibody comprises a glycosylated constant region. In some embodiments, the antibody comprises a constant region having increased binding affinity to one or more human Fc gamma receptor(s).

CD20 is a protein also known as MS4A1, B1, B-lymphocyte antigen CD20, B-lymphocyte surface antigen B1, Bp35, CVID5, LEU-16, Leukocyte surface antigen Leu-16, Membrane-spanning 4-domains subfamily A member 1 , MGC3969, MS4A2, and S7. Human CD20 has the amino acid sequence of:

MTTPRNSVNGTFPAEPMKGPIAMQSGPKPLFRRMSSLVGPTQSFFMRESKT
LGAVQIMNGLFHIALGGLLMIPAGIYAPICVTVWYPLWGGIMYIISGSLLAATEK
NSRKCLVKGKMIMNSLSLFAAISGMILSIMDILNIKISHFLKMESLNFIRAHTPYIN
IYNCEPANPSEKNSPSTQYCYSIQSLFLGILSVMLIFAFFQELVIAGIVENEWKR
TCSRPKSNIVLLSAEEKKEQTIEIKEEVVGLTETSSQPKNEEDIEIIPIQEEEEEE
TETNFPEPPQDQESSPIENDSSP (SEQ ID NO: 1)

Non-limiting examples of antibodies specific for CD20 include: "C2B8" which is now called "Rituximab" (RITUXAN®) (U.S. Pat. No. 5,736,137, incorporated herein by reference), a chimaeric pan-B antibody targeting CD20; the yttrium-[90]-labeled 2B8 murine antibody designated "Y2B8" or "Ibritumomab Tiuxetan" ZEVALIN® (U.S. Pat. No. 5,736,137, incorporated herein by reference), a murine lgG1 kappa mAb covalently linked to MX-DTPA for chelating to yttrium-[90]; murine IgG2a "Bl," also called "Tositumomab," optionally labeled with radioactive 1311 to generate the "131I-B1" antibody (iodine 131 tositumomab, BEXXAR™) (U.S. Pat. No. 5,595,721, expressly incorporated herein by reference); murine monoclonal antibody "1 F5" (Press et al. Blood 69 (2):584-591 (1987) and variants thereof including "framework patched" or humanized 1 F5 (W 003/002607, Leung, S.; ATCC deposit HB-96450); murine 2H7 and chimeric 2H7 antibody (U.S. Pat. No. 5,677,180, expressly incorporated herein by reference); humanized 2H7, also known as ocrelizumab (PRO-70769); Ofatumumab (Arzerra), a fully human IgG1 against a novel epitope on CD20 huMax-CD20 (Genmab, Denmark; WO2004/035607 (U.S. 10/687,799, expressly incorporated herein by reference)); AME-133 (ocaratuzumab; Applied Molecular Evolution), a fully-humanized and optimized IgG1 mAb against CD20; A20 antibody or variants thereof such as chimeric or humanized A20 antibody (cA20, hA20, respectively) (U.S. 10/366,709, expressly incorporated herein by reference, Immunomedics); and monoclonal antibodies L27, G28-2, 93-1 B3, B-CI or NU-B2 available from the International Leukocyte Typing Workshop (Valentine et al, In: Leukocyte Typing III (McMichael, Ed., p. 440, Oxford University Press (1987)). Further, suitable antibodies include, e.g., antibody GA101 (obinutuzumab, GAZYVA®), a third generation humanized anti-CD20-antibody of Biogen Idec/Genentech/Roche. Moreover, BLX-301 of Biolex Therapeutics, a humanized anti CD20 with optimized glycosylation or Veltuzumab (hA20), a 2nd-generation humanized anti-CD20 antibody of Immunomedics or DXL625, derivatives of veltuzumab, such as the bispecific hexavalent antibodies of IBC Pharmaceuticals (Immunomedics) which are comprised of a divalent anti-CD20 IgG of veltuzumab and a pair of stabilized dimers of Fab derived from milatuzumab, an anti-CD20 mAb enhanced with InNexus' Dynamic Cross Linking technology, of Inexus Biotechnology both are humanized anti-CD20 antibodies are suitable. Further suitable antibodies are BM-ca (a humanized anti-CD20 antibody (Int J Oncol. 2011 Feb;38(2):335-44)), C2H7 (a chimeric anti-CD20 antibody (Mol Immunol. 2008 May;45(10):2861 -8)), PRO131921 (a third generation anti-CD20 antibody developed by Genentech), Reditux (a biosimilar version of rituximab developed by Dr Reddy's), PBO-326 (a biosimilar version of rituximab developed by Probiomed), a biosimilar version of rituximab developed by Zenotech, TL-01 1 (a biosimilar version of rituximab developed by Teva), CMAB304 (a biosimilar version of rituximab developed by Shanghai CP Guojian), GP-2013 (a biosimilar version of rituximab developed by Sandoz (Novartis)), SAIT-101 (a biosimilar version of rituximab developed by Samsung BioLogics), a biosimilar version of rituximab developed by Intas Biopharmaceuticals, CT-P10 (a biosimilar version of rituximab developed by Celltrion), a biosimilar version of rituximab developed by Biocad, Ublituximab (LFB-R603, a transgenically produced mAb targeting CD20 developed by GTC Biotherapeutics (LFB Biotechnologies)), PF-05280586 (presumed to be a biosimilar version of rituximab developed by Pfizer), Lymphomun (Bi-20, a trifunctional anti-CD20 and anti-CD3 antibody, developed by Trion Pharma), a biosimilar version of rituximab developed by Natco Pharma, a biosimilar version of rituximab developed by iBio, a biosimilar version of rituximab developed by Gedeon Richter/Stada, a biosimilar version of rituximab developed by Curaxys, a biosimilar version of rituximab developed by Coherus Biosciences/Daiichi Sankyo, a biosimilar version of rituximab developed by BioXpress, BT-D004 (a biosimilar version of rituximab developed by Protheon), AP-052 (a biosimilar version of rituximab developed by Aprogen), a biosimilar version of ofatumumab developed by BioXpress, MG-1 106 (a biosimilar version of rituximab developed by Green Cross), IBI-301 (a humanized monoclonal antibody against CD20 developed by Innovent Biologies), BVX-20 (a humanized mAb against the CD20 developed by Vaccinex), 20-C2-2b (a bispecific mAb-IFNalpha that targets CD20 and human leukocyte antigen-DR (HLA-DR) developed by Immunomedics), MEDI-552 (developed by Medlmmune/AstraZeneca), the anti-CD20/streptavidin conjugates developed by NeoRx (now Poniard Pharmaceuticals), the 2nd generation anti-CD20 human antibodies developed by Favrille (now MMRGIobal), TRU-015, an anti-CD20 antibody fragment developed by Trubion/Emergent BioSolutions, as well as other preclinical approaches by various companies and entities. All of the above-mentioned antibodies are known in the art and/or commercially available. All aforementioned publications, references, patents and patent applications are incorporated by reference in their entireties. All antibodies disclosed in therein may be used within the present disclosure.

In some embodiments, the anti-CD20 antibody is rituximab.

B cell depletion, e.g., using an anti-CD20 antibody, may be accompanied by administration of IVIG to provide the subject with a pool of antibodies to compensate for the loss of B cell protection to the subject. In some embodiments, IVIG is pooled, polyvalent, IgG antibodies extracted from plasma healthy blood donors. Methods for producing IVIG are known in the art (see, e.g., Immune Deficiency Foundation.IDF Patient and Family Handbook: For Primary Immunodeficiency. Disease, 4th Edition. Towson, MD. 2007). IVIG is also commercially available (see, e.g., GAMMAGARD LIQUID® and GAMMAGARD S/D® from Baxter Healthcare, GAMMAPLEX® from Bio Products Laboratory, FLEBOGAMMA® from Grifols, OCTAGAM® from Octapharma, PRIVIGEN® from CSL Behring, GAMUNEX® from Talecris Biotherapeutics, GAMMAKED® from Kedrion and BIVIGAM® from Biotest).

### mTOR pathway inhibitor

Aspects of the disclosure relate to mTOR pathway inhibitors for use in a method or kit described herein. Mammalian target of rapamycin (mTOR) is a serine/threonine kinase, which belongs to phosphatidylinositol-3 kinase (PI3K) related kinases (PIKKs) family. mTOR forms two complexes, mTOR Complex 1 and 2 (mTORC1 and mTORC2). mTORC1 is composed of mTOR, regulatory-associated protein of mTOR (Raptor), mammalian lethal with SEC 13 protein 8 (MLST8) and the non-core components PRAS40 and DEPTOR. mTORC2 is composed of mTOR, rapamycin-insensitive companion of mTOR (RICTOR), MLST8, and mammalian stress-activated protein kinase interacting protein 1 (mSIN1). Accordingly, an mTOR pathway inhibitor may be an inhibitor that binds to and inhibits the activity of mTORC1 and/or mTORC2. Exemplary mTOR pathway inhibitors include, but are not limited to, rapamycin (also known as sirolimus, Pfizer and Wyeth Pharmaceuticals) and other rapalogs including, but not limited to, deforolimus (also known as Ridaforolimus, AP23573, MK-8669, Merck, ARIAD Pharmaceuticals), everolimus (RAD001, Novartis), and temsirolimus (CCI-779, Wyeth Pharmaceuticals). These exemplary mTOR pathway inhibitors, as well as methods of making such inhibitors, are known in the art and are commercially available.

In some embodiments, the mTOR pathway inhibitor is selected from the group consisting of rapamycin, deforolimus, everolimus, and temsirolimus. In some embodiments, the mTOR pathway inhibitor is rapamycin.

### Compositions and methods of administration

Aspects of the disclosure relate to compositions and methods of administration of such compositions. In some embodiments, a composition comprises a rAAV vector described herein for use in a method or kit described herein. In some embodiments, a composition comprises an anti-CD20 antibody described herein (e.g., rituximab) for use in a method or kit described herein. In some embodiments, a composition comprises a mTOR pathway inhibitor described herein (e.g., rapamycin) for use in a method or kit described herein. In some embodiments, a composition comprises IVIG described herein for use in a method or kit described herein. The composition may further comprise a pharmaceutically acceptable carrier. Non-limiting examples of pharmaceutically acceptable carriers include lactose, dextrose, sucrose, sorbitol, mannitol, starches, gum acacia, calcium phosphate, alginates, tragacanth, gelatin, calcium silicate, microcrystalline cellulose, polyvinylpyrrolidone, cellulose, water, saline, syrup, methylcellulose, ethylcellulose, hydroxypropylmethylcellulose, polyacrylic acids, lubricating agents (such as talc, magnesium stearate, and mineral oil), wetting agents, emulsifying agents, suspending agents, preserving agents (such as methyl-, ethyl-, and propyl-hydroxy-benzoates), pH adjusting agents (such as inorganic and organic acids and bases), sweetening agents, and flavoring agents.

The compositions described above may be administered to subject in any suitable formulation by any suitable method. The route of administration of the composition may be oral, parenteral, by inhalation or topical. The term parenteral as used herein includes intravenous, intraarterial, intraperitoneal, intramuscular, intradermal, intrathoracic, intrathecal, and subcutaneous administration.

The compositions described above are typically administered to a subject in an effective amount, that is, an amount capable of producing a desirable result. The desirable result will depend upon the active agent being administered. For example, an effective amount of a rAAV vector may be an amount of the vector that is capable of transferring a heterologous nucleic acid to a host organ, tissue, or cell. An effective amount of an anti-CD20 antibody may be an amount of the antibody that is capable of depleting B cells in a subject (e.g., sufficiently to allow one or more subsequent administrations of a rAAV to a subject). An effective amount of a mTOR pathway inhibitor may be an amount of the inhibitor that is capable of preventing or attenuating a T cell response.

Toxicity and efficacy of the compositions utilized in methods and kits of the disclosure can be determined by standard pharmaceutical procedures, using either cells in culture or experimental animals to determine the LD50 (the dose lethal to 50% of the population). The dose ratio between toxicity and efficacy is the therapeutic index and it can be expressed as the ratio LD50/ED50. Those compositions that exhibit large therapeutic indices are preferred. While those that exhibit toxic side effects may be used, care should be taken to design a delivery system that minimizes the potential damage of such side effects. The dosage of compositions as described herein lies generally within a range that includes an ED50 with little or no toxicity. The dosage may vary within this range depending upon the dosage form employed and the route of administration utilized.

As is well known in the medical and veterinary arts, dosage for any one subject depends on many factors, including the subject's size, body surface area, age, the particular composition to be administered, the active ingredient(s) in the composition, time and route of administration, general health, and other drugs being administered concurrently. In some embodiments, an appropriate dosage for intravenous administration of a rAAV vector may be in the range of about 10¹²-10¹⁵ vectors. In some embodiments, an appropriate dosage for intravenous administration of an anti-CD20 antibody (e.g., rituximab) may be 375 mg/m². In some embodiments, the anti-CD20 antibody is administered weekly or once every 12 weeks. In some embodiments, an appropriate dosage for oral rapamycin may be 0.06-1 mg/m². In some embodiments, an appropriate dosage for oral rapamycin is a dosage that maintains a serum trough level of 3-7 ng/ml. In some embodiments, the rapamycin is administered daily. In some embodiments, an appropriate dosage for IVIG is 500-1000 mg/kg of IVIG. In some embodiments, an appropriate dosage for IVIG is a dosage that maintains a trough serum IgG level of 700-1000 mg/dL.

In some embodiments, compositions comprising rAAV vectors may be directly introduced into a subject, including by intravenous (IV) injection, intraperitoneal (IP) injection, or in situ injection into target tissue (e.g., muscle). For example, a conventional syringe and needle can be used to inject a rAAV vector suspension into an animal. Depending on the desired route of administration, injection can be in situ (i.e., to a particular tissue or location on a tissue), intramuscular, IV, IP, or by another parenteral route. Parenteral administration of rAAV vectors by injection can be performed, for example, by bolus injection or continuous infusion. Formulations for injection may be presented in unit dosage form, for example, in ampoules or in multi-dose containers, with an added preservative. The compositions may take such forms as suspensions, solutions or emulsions in oily or aqueous vehicles, and may contain formulatory agents such as suspending, stabilizing and/or dispersing agents. Alternatively, the rAAV vectors may be in powder form (e.g., lyophilized) for constitution with a suitable vehicle, for example, sterile pyrogen-free water, before use.

To facilitate delivery of the composition comprising the rAAV vector to a subject, the rAAV vector of the invention can be mixed with a carrier or excipient. Carriers and excipients that might be used include saline (e.g., sterilized, pyrogen-free saline) saline buffers (e.g., citrate buffer, phosphate buffer, acetate buffer, and bicarbonate buffer), amino acids, urea, alcohols, ascorbic acid, phospholipids, proteins (for example, serum albumin), EDTA, sodium chloride, liposomes, mannitol, sorbitol, and glycerol. USP grade carriers and excipients are particularly useful for delivery of rAAV vectors to human subjects. Methods for making such formulations are well known and can be found in, for example, Remington's Pharmaceutical Sciences.

In addition to the formulations described previously, the rAAV vectors can also be formulated as a depot preparation. Such long acting formulations may be administered by implantation (for example subcutaneously or intramuscularly) or by intramuscular (IM) injection. Thus, for example, the rAAV vector may be formulated with suitable polymeric or hydrophobic materials (for example as an emulsion in an acceptable oil) or ion exchange resins, or as sparingly soluble derivatives.

In some embodiments, compositions comprising an anti-CD20 antibody or IVIG may be administered by parenteral administration. The intravenous, intraarterial, subcutaneous and intramuscular forms of parenteral administration are generally preferred. Usually, a suitable pharmaceutical composition for parenteral injection may comprise a buffer (e.g., acetate, phosphate or citrate buffer), a surfactant (e.g., polysorbate), optionally a stabilizer agent (e.g., human albumine), etc. Exemplary preparations for parenteral administration of the anti-CD20 antibody include sterile aqueous or non-aqueous solutions, suspensions, and emulsions. Examples of non-aqueous solvents are propylene glycol, polyethylene glycol, vegetable oils such as olive oil, and injectable organic esters such as ethyl oleate. Aqueous carriers include water, alcoholic/aqueous solutions, emulsions or suspensions, including saline and buffered media. In the subject invention, pharmaceutically acceptable carriers include, but are not limited to, 0.01-0.1M and preferably 0.05M phosphate buffer or 0.8% saline. Other common parenteral vehicles include sodium phosphate solutions, Ringer's dextrose, dextrose and sodium chloride, lactated Ringer's, or fixed oils. Intravenous vehicles include fluid and nutrient replenishers, electrolyte replenishers, such as those based on Ringer's dextrose, and the like. Preservatives and other additives may also be present such as for example, antimicrobials, antioxidants, chelating agents, and inert gases and the like.

More particularly, pharmaceutical compositions suitable for injectable use include sterile aqueous solutions (where water soluble) or dispersions and sterile powders for the extemporaneous preparation of sterile injectable solutions or dispersions. In such cases, the composition must be sterile and should be fluid to the extent that easy syringability exists. It should be stable under the conditions of manufacture and storage and will preferably be preserved against the contaminating action of microorganisms, such as bacteria and fungi. The carrier can be a solvent or dispersion medium containing, for example, water, ethanol, polyol (e.g., glycerol, propylene glycol, and liquid polyethylene glycol, and the like), and suitable mixtures thereof. The proper fluidity can be maintained, for example, by the use of a coating such as lecithin, by the maintenance of the required particle size in the case of dispersion and by the use of surfactants.

A sterile injectable solutions can be prepared by incorporating an active compound (e.g., an anti-CD20 antibody or IVIG) in the required amount in an appropriate solvent with one or a combination of ingredients enumerated herein, as required, followed by filtered sterilization. Generally, dispersions are prepared by incorporating the active compound into a sterile vehicle, which contains a basic dispersion medium and the required other ingredients from those enumerated above. In the case of sterile powders for the preparation of sterile injectable solutions, the preferred methods of preparation are vacuum drying and freeze-drying, which yields a powder of an active ingredient plus any additional desired ingredient from a previously sterile-filtered solution thereof. The preparations for injections are processed, filled into containers such as ampoules, bags, bottles, syringes or vials, and sealed under aseptic conditions according to methods known in the art.

In some embodiments, the mTOR pathway inhibitor is administered by oral or parenteral administration. The pharmaceutical carrier for administration may be solid or liquid. A solid carrier can include one or more substances which may also act as flavoring agents, lubricants, solubilizers, suspending agents, fillers, glidants, compression aids, binders or tablet-disintegrating agents; it can also be an encapsulating material. In powders, the carrier is a finely divided solid which is in admixture with the finely divided active ingredient. In tablets, the active ingredient is mixed with a carrier having the necessary compression properties in suitable proportions and compacted in the shape and size desired. The powders and tablets preferably contain up to 99% of the active ingredient. Suitable solid carriers include, for example, calcium phosphate, magnesium stearate, talc, sugars, lactose, dextrin, starch, gelatin, cellulose, methyl cellulose, sodium carboxymethyl cellulose, polyvinylpyrrolidine, low melting waxes and ion exchange resins.

Liquid carriers may be used in preparing solutions, suspensions, emulsions, syrups, elixirs and pressurized compositions. The active ingredient can be dissolved or suspended in a pharmaceutically acceptable liquid carrier such as water, an organic solvent, a mixture of both or pharmaceutically acceptable oils or fats. The liquid carrier can contain other suitable pharmaceutical additives such as solubilizers, emulsifiers, buffers, preservatives, sweeteners, flavoring agents, suspending agents, thickening agents, colors, viscosity regulators, stabilizers or osmo-regulators. Suitable examples of liquid carriers for oral and parenteral administration include water (partially containing additives as above, e.g., cellulose derivatives, preferably sodium carboxymethyl cellulose solution), alcohols (including monohydric alcohols and polyhydric alcohols, e.g., glycols) and their derivatives, and oils (e.g., fractionated coconut oil and arachis oil). For parenteral administration, the carrier can also be an oily ester such as ethyl oleate and isopropyl myristare. Sterile liquid carriers are useful in sterile liquid form compositions for parenteral administration. The liquid carrier for pressurized compositions can be halogenated hydrocarbon or other pharmaceutically acceptable propellent.

Liquid pharmaceutical compositions comprising a mTOR pathway inhibitor which are sterile solutions or suspensions can be utilized by, for example, intramuscular, intraperitoneal or subcutaneous injection. Sterile solutions can also be administered intravenously. The mTOR pathway inhibitor can also be administered orally either in liquid or solid composition form.

### Kits

Aspects of the disclosure relate to kits. In some embodiments the kit, comprises (a) a rAAV vector, (b) an anti-CD20 antibody described herein; and (c) an mTOR pathway inhibitor described herein. In some embodiments, the anti-CD20 antibody is rituximab. In some embodiments, the mTOR pathway inhibitor is rapamycin. Any of the components may be comprised within a composition described herein, e.g., a composition comprising a pharmaceutically acceptable carrier. In some embodiments, the kit further comprises a syringe. In some embodiments, the kit further comprises instructions for use, e.g., instructions describing a method provided herein. The instructions may be paper instructions or instructions in an electronic format.

Without further elaboration, it is believed that one skilled in the art can, based on the above description, utilize the present disclosure to its fullest extent. The following specific embodiments are, therefore, to be construed as merely illustrative, and not limitative of the remainder of the disclosure in any way whatsoever. All publications cited herein are incorporated by reference for the purposes or subject matter referenced herein.

### EXAMPLES

### Example 1: B-cell depletion is Protective Against Anti-AAV Capsid Immune Response Introduction

Pompe disease is a progressive and often fatal neuromuscular disorder resulting from mutation in the gene for acid alpha-glucosidase (GAA), an enzyme necessary for the degradation of lysosomal glycogen within the lysosome. The condition is characterized by a spectrum of disease severity resulting from variable levels of GAA and possibly differential cellular rates of glycogen synthesis. The result of GAA deficiency is an extensive glycogen accumulation in all tissues, especially striated muscle, smooth muscle and the central nervous system (CNS) (refs. 1-3). The range of disease severity encompasses the fatal early-onset form presenting in infancy to a milder adult-onset of disease symptoms. The disease prevalence has been estimated to be 2-4,000 patients in the developed world and incidence is approximately 1 per 40,000 births (refs. 4,5). The phenotypic continuum is directly related to the extent of residual enzyme deficiency (refs. 2,6) with complete or near complete deficiency of functional GAA protein in early-onset disease and up to 20% of wild-type activity in late-onset patients (refs. 7,8).

Respiratory and skeletal muscle weakness is a key progressive feature of Pompe disease. Respiratory muscle weakness often leads to the need for assisted ventilation and is the principal cause of mortality in early and late onset Pompe patients (refs. 9,10). Skeletal muscle weakness primarily affects the lower limbs and results in loss of ambulation and wheelchair dependency (ref. 11). Generally, GAA activity <1% of wild-type level correlates with presentation in infancy, and 2%-20% GAA activity is seen in later-onset disease (ref. 4,5). Approximately 25% of infants with <1% GAA activity have no detectable GAA protein by Western blot analysis and are considered cross-reactive immunologic material (CRIM)-negative (ref. 12). In CRIM-positive patients, the presence of residual GAA protein usually correlates with a lack of neutralizing antibodies (Nabs) against GAA after initiation of enzyme replacement therapy (ERT). In contrast, CRIM-negative patients lack tolerance to GAA protein and produce a robust humoral immune response to ERT, reducing the efficacy of treatment. CRIM-negative patients receiving ERT have a poor prognosis and diminished survival if not managed with immunosuppression (refs. 13-16).

ERT has extended the survival for early onset patients, however, a successful gene therapy strategy may provide additional long-term benefits and improvement in quality of life. Recombinant adeno-associated viral vectors (rAAV) are widely used gene therapy agents for the treatment of genetic diseases. The success of rAAV is based on its safety profile, the ability to transduce both dividing and non-dividing cells and in its low immunogenicity. AAV has been used in several clinical trials for the treatment of different conditions including Leber's congenital amaurosis (refs. 17,18), hemophilia B (refs. 19,20), Pompe disease (ref. 21), San Filippo syndrome (ref. 22), lipoprotein lipase deficiency (refs. 23,24), Alpha-1 antitrypsin deficiency (ref. 25), and Limb-girdle muscular dystrophy (refs. 26,27). A critical challenge for the success of gene therapy is the host immune responses to both the vector capsid and transgene product. These immune responses raise concerns regarding the safety and longevity of gene expression. In addition, induction of antibodies by natural exposure to AAV is frequent early in life and may influence the use of AAV as a gene therapy vector (refs. 28,29). This may be critical in developing effective therapeutic strategies for congenital myopathies that may require repeat administration of AAV vectors. Repeat AAV administration may be necessary because low doses, or doses below optimal therapeutic threshold were provided in early phase studies. Further, many subjects may require re-dosing later in life, as increasing muscle mass or loss of copy number with age may reduce transgene expression. Therefore, potent humoral and cellular memory responses to AAV may compromise the subsequent use of the same vector (refs. 28,29). For these reasons, strategies are being developed to manage these immune responses as a sustainable approach to deliver a safe and long-term expression of a therapeutic gene by AAV-mediated gene therapy.

Human immune responses to foreign antigens include humoral and cellular responses to the AAV capsid and to the transgene product. Humoral responses to ERT observed in Pompe disease include activation of antigen-specific CD4⁺ T helper cells and production of neutralizing (NAbs) and non-neutralizing (non-NAbs) antibodies. NAbs have been the focus of gene therapy immunology studies because of their destructive effect on the efficacy of AAV-mediated gene therapy. NAbs bind to the AAV capsid and may block or reduce the transduction of target cells. Additionally, anti-transgene antibodies may develop against the therapeutic protein or may serve as co-activating factors for cell-mediated immunity, possibly leading to elimination of transduced cells (refs. 28, 29).

One of the strategies to overcome the presence of Nabs in rAAV-mediated gene therapy is pharmacological modulation of the humoral immune response. Rituximab is a monoclonal antibody that induces B-cell depletion by binding the CD20 protein found on the surface of B-cells. Rituximab is FDA approved in the treatment of chronic lymphocytic leukemia, non-Hodgkin's lymphoma, and rheumatoid arthritis. Recent studies also showed that Rituximab is able to improve immune tolerance induction in patients with hemophilia (refs. 30-32). In addition, Rituximab was successful in reduction of existing anti-rAAV NAbs in humans receiving rituximab for rheumatoid arthritis (ref. 33) and in non-human primates (refs. 19, 34). Rituximab's mechanism of action includes complement-mediated cell lysis, antibody-dependent cell-mediated cytotoxicity and direct induction of apoptosis by binding to CD20 (ref. 35).

Rituximab may be used in combination with a B-cell and T-cell agent, such as Sirolimus (ref. 36). Sirolimus inhibits the response to interleukin-2 by binding the FK-binding protein 12 and the Sirolimus-FKBP12 complex acts as an inhibitor of the mammalian target of rapamycin (mTOR) pathway and has been used in preclinical studies for gene therapy (refs. 37-39).

Elder et al. (refs. 13,15) showed that an immune suppression protocol based on B-cell depletion with Rituximab and T-cell immunomodulation with Sirolimus before initiation of ERT in CRIM-negative infants with early-onset Pompe disease was able to block GAA antibody responses and improve clinical outcomes. Described herein are the immunological and clinical consequences of B-cell depletion and T-cell immunomodulation in a cross-reacting immunologic material-negative (CRIM-) child with Pompe disease, who was receiving Rituximab and Sirolimus prior to and during enrollment of a phase I-II clinical trial of intramuscular administration of an rAAV1-CMV-hGAA vector protocol (refs. 21). It is shown that an immunomodulatory regimen with Rituximab and Sirolimus may be beneficial to control expression of immune responses following gene therapy approaches in humans.

### Results

### Subject characteristics

The subject of this case study was a CRIM- female child who was diagnosed with Pompe disease (nonsense mutation exon 10, p.W516X; exon 18, p.G828_N882del) at 5.5 months of age. CRIM status was evaluated at the Powell Gene Therapy Center (Gainesville, Florida) by Western blot analysis of skin fibroblasts. The patient presented a severe phenotype with GAA activity <1%, cardiac hypertrophy and ultimately required assisted ventilation. Immediately after diagnosis she was enrolled in a study for which she received ERT and immune modulation for management of antibody response as part of the prescribed treatment regimen. Details of this study are presented in Elder et al. (ref. 13). At 45 months of age, the patient was enrolled in another study evaluating the effect of rAAV1-CMV-hGAA intramuscular gene transfer to the diaphragm. Details of this study are presented in Smith et al. (ref. 21). FIG. 1 summarizes the events of this case study.

### Immunosuppression protocol and enzyme replacement therapy (ERT)

At 5.5 months of age, about 20 days before starting ERT, the patient received 375 mg/m² of rituximab and 10mg/kg of methylprednisolone intravenously (premedication) weekly for 3 weeks. After the Rituximab induction doses, the patient received daily oral Sirolimus (0.06-1 mg/m²/day). The dosage was adjusted in order to maintain a Sirolimus serum trough level of 3-7 ng/ml. Once the induction dosage of Rituximab was completed, the patient started receiving a monthly dose of 500-1000 mg/kg of IV immunoglobulin (IVIG) to replace the IgG pool of the patient that is lost upon B cell depletion (GAMUNEX®; Talecris Biotherapeutics, Research Triangle Park, North Carolina or PRIVIGEN®; CSL Behring, King of Prussia, Pennsylvania). The amount was adjusted in order to maintain a trough serum IgG level of 700-1000 mg/dL. The patient continued B-cell depletion regimen with Rituximab at 12 weeks intervals while receiving Sirolimus daily. At 6 months of age, after initiation of B cell depletion, the patient started on ERT infusion at a dose of 20 mg/kg every 7-10 days. The ERT infusion interval was subsequently increased to every 10 - 14 days once clinical improvement was demonstrated.

### rAAV-mediated gene therapy

The patient was enrolled in a Phase I/II study to determine the safety and ventilatory outcomes following intramuscular administration of rAAV1-CMV-hGAA (NCT00976352; ClincalTrials.gov). AAV dosing was accomplished at 45 months of age via bilateral diaphragmatic delivery using video-assisted thoracoscopy. The vector was injected in each hemidiaphragm in three sites, corresponding to the anterior, lateral and posterior costal regions. Each site received 8.33 x 10¹¹ vg in 0.8 ml. The total dose was 5 x 10¹² vg of rAAV1-CMV-hGAA 21.

### Anti-rAAV1 and anti-hGAA circulating antibody and T-cell-mediated responses

Serum samples were assayed by ELISA for circulating antibodies to the AAV1 capsid proteins. The case patient was compared to an immune-modulation control group, which consisted of five children with Pompe disease that received 1-5 x 10¹² vg/kg of rAAV1-CMV-hGAA but did not receive the immunosuppression regimen. The control group developed at least a 155-fold increase in anti-AAV1 Ab titer post exposure to AAV1. The patient of this report had no response to AAV capsids through day 180 (FIG. 2). The baseline and subsequent titers were at least 2 logs above the limit of detection for the antibody assay.

Enzyme linked immunospot (ELISpot) assay and antigen specific response assay (ASR) were used to test CD4⁺ and CD8⁺ T-cells for reactivity against both the AAV capsid and the transgene products. ELISPOT assays to AAV1 capsid protein peptide pools and ASR (FIG. 2) to intact AAV1 capsids were unchanged from baseline to day 180. Together, these findings demonstrate the potential to block humoral and cellular immune response following exposure to AAV1. The described regimen facilitated successful regional gene transfer with no adverse events and resulted in a clinical benefit.

### Clinical pathology laboratory evaluation.

Safety laboratory data is shown in FIG. 3. Serum IgG was maintained above 700-1000 mg/dL by regular monthly infusions of IVIG. The approach had no impact on WBC, nor was there any evidence of thrombocytopenia or anemia. Serum transaminase levels are commonly elevated in Pompe disease (ref. 40) and were unchanged in the patient, from baseline through the 90 days following rAAV1-CMV-hGAA dosing. NT-pro BNP, CPK and CPK-MB were responsive to initiation of ERT and were not influenced by the immune management. There was no impact on CD3 positive lymphocytes. Evaluation of CD20 counts confirmed complete depletion of B-cells by Rituximab.

There were no adverse events identified during the follow up period to date related to the study procedure or immunomodulation regimen.

### Functional measures

Maximal Inspiratory Pressure (MIP) remained stable at day 90 and increased 6% of baseline value at day 180. Motor Function Measure (GMFM-88) increased 2.95% at day 90 and decreased 1.33% at day 180 (FIG. 4), which are not clinically significant improvements.

It was observed that B-cell depletion with rituximab prior to rAAV vector exposure resulted in immune non-responsiveness to rAAV-CMV-hGAA vector administration, therefore allowing for the possibility of repeat administration of the a vector, e.g., a vector of the same AAV serotype. Accordingly, methods involving administration of rituximab are useful for permitting a subject to be administered multiple doses of rAAV. This approach can be applied to prevent immune response to rAAV vectors administered systemically or targeted to cardiac and skeletal muscles or liver.

This approach could be useful in any subject, including pediatric subjects who will be likely candidates for multiple dosing events over the course of their lifetime. Three basic principles support this conclusion. First, the body mass in a pediatric patient will increase up to 20-fold from infancy to adulthood, and the dilution of vector genomes would be expected to be greater than 2-log change accounting for cellular turnover, even in muscle and neural tissues. Second, many of the conditions for which early rAAV-mediated gene therapy is being considered have increased rates of cell division or cell death due to the underlying disease. Either of these factors alone or in combination will almost certainly lead to a decline in genome copy number per cell, leading to loss of efficacy over time. Third, early phase studies where subjects in low-dose cohorts would not receive a clinically effective dose may impact on the need for re-exposure to the therapeutic vector. In order to ethically manage such studies in rare disease populations, it is appropriate to have a strategy in hand to remove the confounding effects of anti-capsid immunity and allow such subjects the chance to enter subsequent studies where a more effective dose is being proposed.

This study was approved by the Institutional Review Board at the University of Florida and all procedures were performed in consideration of the Declaration of Helsinki. All patients gave their written informed consent before participating to this study. The study is registered at clinicaltrials.gov, identifier NCT00976352.

### Outcome measures

### Safety measures

Laboratory data and clinical evaluation confirmed safety of the procedure. Other safety laboratory studies included quantitative real-time polymerase chain reaction (PCR) to assess the presence of vector DNA in peripheral blood. Serum samples were assayed by enzyme linked immunosorbent assay (ELISA) for circulating antibodies to the intact AAV1 capsid and hGAA protein 21,46. Anti-AAV1 and anti-hGAA ASR and ELISPOT assay were performed as previously described on isolated peripheral blood mononuclear cells (ref. 47). Enumeration of B- and T-lymphocyte subpopulations in peripheral blood was done by flow cytometry using monoclonal Abs to CD19 or CD20 for B cells and monoclonal Abs to CD3, CD4, and CD8 for T cells. Sirolimus trough levels, IgG, CD3, CD4, CD8, CD19, CD20, N-terminal pro-brain natriuretic peptide, creatine kinase, creatine kinase-MB, C-reactive protein, platelets, alkaline phosphatase, gamma-glutamyl transferase, aspartate aminotransferase, and alanine aminotransferase were measured at regular intervals.

### Functional measures

MIP was evaluated using a one-way inspiratory occlusion. A one-way valve permitted exhalation, but prevented inspiratory airflow. A pressure transducer was used to record the negative pressure. The test was repeated three times and the most negative pressure was recorded.

GMFM-88 (ref. 48), which is an evaluative tool designed to measure change in the gross motor function of patients over time (e.g., lying/rolling, sitting, crawling/kneeling, standing and walking/running/jumping) was also tested. MIP and GMFM-88 were collected at baseline, day 90 and day 180.

### References

1. DeRuisseau, L.R. et al. Neural deficits contribute to respiratory insufficiency in Pompe disease. Proc Natl Acad Sci U S A 106, 9419-24 (2009).
2. Raben, N., Plotz, P. & Byrne, B.J. Acid alpha-glucosidase deficiency (glycogenosis type II, Pompe disease). Curr Mol Med 2, 145-66 (2002).
3. Sidman, R.L. et al. Temporal neuropathologic and behavioral phenotype of 6neo/6neo Pompe disease mice. J Neuropathol Exp Neurol 67, 803-18 (2008).
4. Byrne, B.J. et al. Pompe disease gene therapy. Hum Mol Genet 20, R61-8 (2011).
5. Byrne, B.J. et al. Pompe disease: design, methodology, and early findings from the Pompe Registry. Mol Genet Metab 103, 1-11 (2011).
6. Hirschhorn, J.N. et al. SBE-TAGS: an array-based method for efficient single-nucleotide polymorphism genotyping. Proc Natl Acad Sci U S A 97, 12164-9 (2000).
7. Toscano, A. & Schoser, B. Enzyme replacement therapy in late-onset Pompe disease: a systematic literature review. J Neurol 260, 951-9 (2013).
8. van der Beek, N.A., Hagemans, M.L., van der Ploeg, A.T., Reuser, A.J. & van Doom, P.A. Pompe disease (glycogen storage disease type II): clinical features and enzyme replacement therapy. Acta Neurol Belg 106, 82-6 (2006).
9. Moufarrej, N.A. & Bertorini, T.E. Respiratory insufficiency in adult-type acid maltase deficiency. South Med J 86, 560-7 (1993).
10. McKusick, V.A., Amberger, J.S. & Francomano, C.A. Progress in medical genetics: map-based gene discovery and the molecular pathology of skeletal dysplasias. Am J Med Genet 63, 98-105 (1996).
11. Leslie, N. & Tinkle, B.T. Glycogen Storage Disease Type II (Pompe Disease). in GeneReviews (eds. Pagon, R.A. et al.) (Seattle (WA), 1993).
12. Bali, D.S., Tolun, A.A., Goldstein, J.L., Dai, J. & Kishnani, P.S. Molecular analysis and protein processing in late-onset Pompe disease patients with low levels of acid alpha-glucosidase activity. Muscle Nerve 43, 665-70 (2011).
13. Elder, M.E. et al. B-Cell depletion and immunomodulation before initiation of enzyme replacement therapy blocks the immune response to acid alpha-glucosidase in infantile-onset Pompe disease. J Pediatr 163, 847-54 e1 (2013).
14. Banugaria, S.G. et al. Persistence of high sustained antibodies to enzyme replacement therapy despite extensive immunomodulatory therapy in an infant with Pompe disease: need for agents to target antibody-secreting plasma cells. Mol Genet Metab 105, 677-80 (2012).
15. Banugaria, S.G. et al. Algorithm for the early diagnosis and treatment of patients with cross reactive immunologic material-negative classic infantile pompe disease: a step towards improving the efficacy of ERT. PLoS One 8, e67052 (2013).
16. Kishnani, P.S. et al. Cross-reactive immunologic material status affects treatment outcomes in Pompe disease infants. Mol Genet Metab 99, 26-33 (2010).
17. Cideciyan, A.V. et al. Vision 1 year after gene therapy for Leber's congenital amaurosis. N Engl J Med 361, 725-7 (2009).
18. Jacobson, S.G. et al. Gene therapy for leber congenital amaurosis caused by RPE65 mutations: safety and efficacy in 15 children and adults followed up to 3 years. Arch Ophthalmol 130, 9-24 (2012).
19. Nathwani, A.C. et al. Safe and efficient transduction of the liver after peripheral vein infusion of self-complementary AAV vector results in stable therapeutic expression of human FIX in nonhuman primates. Blood 109, 1414-21 (2007).
20. Manno, C.S. et al. Successful transduction of liver in hemophilia by AAV-Factor IX and limitations imposed by the host immune response. Nat Med 12, 342-7 (2006).
21. Smith, B.K. et al. Phase I/II trial of adeno-associated virus-mediated alpha-glucosidase gene therapy to the diaphragm for chronic respiratory failure in Pompe disease: initial safety and ventilatory outcomes. Hum Gene Ther 24, 630-40 (2013).
22. Tardieu, M. et al. Intracerebral administration of AAV rh.10 carrying human SGSH and SUMF1 cDNAs in children with MPSIIIA disease: results of a phase I/II trial. Hum Gene Ther (2014).
23. Ferreira, V. et al. Immune responses to intramuscular administration of alipogene tiparvovec (AAV1-LPLS447X) in a phase II clinical trial of Lipoprotein Lipase deficiency (LPLD) gene therapy. Hum Gene Ther (2013).
24. Kaeppel, C. et al. A largely random AAV integration profile after LPLD gene therapy. Nat Med 19, 889-91 (2013).
25. Brantly, M.L. et al. Phase I trial of intramuscular injection of a recombinant adeno-associated virus serotype 2 alphal-antitrypsin (AAT) vector in AAT-deficient adults. Hum Gene Ther 17, 1177-86 (2006).
26. Mendell, J.R. et al. Limb-girdle muscular dystrophy type 2D gene therapy restores alpha-sarcoglycan and associated proteins. Ann Neurol 66, 290-7 (2009).
27. Rodino-Klapac, L.R., Lee, J.S., Mulligan, R.C., Clark, K.R. & Mendell, J.R. Lack of toxicity of alpha-sarcoglycan overexpression supports clinical gene transfer trial in LGMD2D. Neurology 71, 240-7 (2008).
28. Calcedo, R. & Wilson, J.M. Humoral Immune Response to AAV. Front Immunol 4, 341 (2013).
29. Mingozzi, F. & High, K.A. Immune responses to AAV vectors: overcoming barriers to successful gene therapy. Blood 122, 23-36 (2013).
30. Aleem, A. et al. Rituximab as a single agent in the management of adult patients with haemophilia A and inhibitors: marked reduction in inhibitor level and clinical improvement in bleeding but failure to eradicate the inhibitor. Haemophilia 15, 210-6 (2009).
31. Collins, P.W. et al. Rituximab and immune tolerance in severe hemophilia A: a consecutive national cohort. J Thromb Haemost 7, 787-94 (2009).
32. Streif, W. et al. Inhibitor treatment by rituximab in congenital haemophilia A - Two case reports. Hamostaseologie 29, 151-4 (2009).
33. Mingozzi, F. et al. Prevalence and pharmacological modulation of humoral immunity to AAV vectors in gene transfer to synovial tissue. Gene Ther 20, 417-24 (2013).
34. Masat, E., Pavani, G. & Mingozzi, F. Humoral immunity to AAV vectors in gene therapy: challenges and potential solutions. Discov Med 15, 379-89 (2013).
35. Weiner, G.J. Rituximab: mechanism of action. Semin Hematol 47, 115-23 (2010).
36. Levitsky, J. et al. Systemic immunoregulatory and proteogenomic effects of tacrolimus to sirolimus conversion in liver transplant recipients. Hepatology 57, 239-48 (2013).
37. Nayak, S. et al. Mapping the T helper cell response to acid alpha-glucosidase in Pompe mice. Mol Genet Metab 106, 189-95 (2012).
38. Nayak, S. et al. Prophylactic immune tolerance induced by changing the ratio of antigen-specific effector to regulatory T cells. J Thromb Haemost 7, 1523-32 (2009).
39. Nayak, S. & Herzog, R.W. Progress and prospects: immune responses to viral vectors. Gene Ther 17, 295-304 (2010).
40. Disease, A.W.G.o.M.o.P. et al. Pompe disease diagnosis and management guideline. Genet Med 8, 267-88 (2006).
41. Mingozzi, F. et al. CD8(+) T-cell responses to adeno-associated virus capsid in humans. Nat Med 13, 419-22 (2007).
42. Li, H. et al. Capsid-specific T-cell responses to natural infections with adeno-associated viruses in humans differ from those of nonhuman primates. Mol Ther 19, 2021-30 (2011).
43. Falk DD, C.D., Nayak S, Doerfler P, Corti M, Clement N, Conlon T, Tarantal AF, Byrne BJ. Prevention of anti-capsid response following systemic AAV9 dosing infant rhesus monkeys. Molecular Therapy 21, e1-e46 (2013).
44. Chicoine, L. et al. Plasmapheresis Eliminates the Negative Impact of AAV Antibodies on Microdystrophin Gene Expression Following Vascular Delivery. Mol Ther (2013).
45. Karman, J. et al. Proteasome inhibition is partially effective in attenuating pre-existing immunity against recombinant adeno-associated viral vectors. PLoS One 7, e34684 (2012).
46. Brantly, M.L. et al. Sustained transgene expression despite T lymphocyte responses in a clinical trial of rAAV1-AAT gene therapy. Proc Natl Acad Sci U S A 106, 16363-8 (2009).
47. Hauswirth, W.W. et al. Treatment of leber congenital amaurosis due to RPE65 mutations by ocular subretinal injection of adeno-associated virus gene vector: short-term results of a phase I trial. Hum Gene Ther 19, 979-90 (2008).
48. Hielkema, T. et al. GMFM in infancy: age-specific limitations and adaptations. Pediatr Phys Ther 25, 168-76; discussion 177 (2013).

### Example 2: Assessment of circulating antibodies against AAV1

Additional subjects were treated with rAAV1-CMV-GAA in combination with an immunosuppression protocol as described in Example 1 (treated with Rituximab and sirolimus) or were treated with rAAV1-CMV-GAA without immunosuppression. Subjects who received 1-5 x 10¹² vg/kg of rAAV1-CMV-GAA without immunosuppression (n=5, lines with circles in FIG. 5), had anti-AAV1 antibodies >2 standard deviations (SD) above the population mean by day 14 post-dosing. In contrast, 3 subjects who received 5 x 10¹² vg/kg of AAV1-CMV1-GAA in conjunction with pre- and post-dosing immunosuppression (lines with triangles, diamonds and squares in FIG. 5) did not generate humoral responses to AAV1. These findings further support the possibility of re-dosing the immunosuppressed subjects with AAV at a subsequent time point.

### OTHER EMBODIMENTS

All of the features disclosed in this specification may be combined in any combination. Each feature disclosed in this specification may be replaced by an alternative feature serving the same, equivalent, or similar purpose. Thus, unless expressly stated otherwise, each feature disclosed is only an example of a generic series of equivalent or similar features.

From the above description, one skilled in the art can easily ascertain the essential characteristics of the present disclosure, and without departing from the spirit and scope thereof, can make various changes and modifications of the disclosure to adapt it to various usages and conditions. Thus, other embodiments are also within the claims.

### EQUIVALENTS

While several inventive embodiments have been described and illustrated herein, those of ordinary skill in the art will readily envision a variety of other means and/or structures for performing the function and/or obtaining the results and/or one or more of the advantages described herein, and each of such variations and/or modifications is deemed to be within the scope of the inventive embodiments described herein. More generally, those skilled in the art will readily appreciate that all parameters, dimensions, materials, and configurations described herein are meant to be exemplary and that the actual parameters, dimensions, materials, and/or configurations will depend upon the specific application or applications for which the inventive teachings is/are used. Those skilled in the art will recognize, or be able to ascertain using no more than routine experimentation, many equivalents to the specific inventive embodiments described herein. It is, therefore, to be understood that the foregoing embodiments are presented by way of example only and that, within the scope of the appended claims and equivalents thereto, inventive embodiments may be practiced otherwise than as specifically described and claimed. Inventive embodiments of the present disclosure are directed to each individual feature, system, article, material, kit, and/or method described herein. In addition, any combination of two or more such features, systems, articles, materials, kits, and/or methods, if such features, systems, articles, materials, kits, and/or methods are not mutually inconsistent, is included within the inventive scope of the present disclosure.

All definitions, as defined and used herein, should be understood to control over dictionary definitions, definitions in documents incorporated by reference, and/or ordinary meanings of the defined terms.

All references, patents and patent applications disclosed herein are incorporated by reference with respect to the subject matter for which each is cited, which in some cases may encompass the entirety of the document.

The indefinite articles "a" and "an," as used herein in the specification and in the claims, unless clearly indicated to the contrary, should be understood to mean "at least one."

The phrase "and/or," as used herein in the specification and in the claims, should be understood to mean "either or both" of the elements so conjoined, i.e., elements that are conjunctively present in some cases and disjunctively present in other cases. Multiple elements listed with "and/or" should be construed in the same fashion, i.e., "one or more" of the elements so conjoined. Other elements may optionally be present other than the elements specifically identified by the "and/or" clause, whether related or unrelated to those elements specifically identified. Thus, as a non-limiting example, a reference to "A and/or B", when used in conjunction with open-ended language such as "comprising" can refer, in one embodiment, to A only (optionally including elements other than B); in another embodiment, to B only (optionally including elements other than A); in yet another embodiment, to both A and B (optionally including other elements); etc.

As used herein in the specification and in the claims, "or" should be understood to have the same meaning as "and/or" as defined above. For example, when separating items in a list, "or" or "and/or" shall be interpreted as being inclusive, i.e., the inclusion of at least one, but also including more than one, of a number or list of elements, and, optionally, additional unlisted items. Only terms clearly indicated to the contrary, such as "only one of" or "exactly one of," or, when used in the claims, "consisting of," will refer to the inclusion of exactly one element of a number or list of elements. In general, the term "or" as used herein shall only be interpreted as indicating exclusive alternatives (i.e. "one or the other but not both") when preceded by terms of exclusivity, such as "either," "one of," "only one of," or "exactly one of." "Consisting essentially of," when used in the claims, shall have its ordinary meaning as used in the field of patent law.

As used herein in the specification and in the claims, the phrase "at least one," in reference to a list of one or more elements, should be understood to mean at least one element selected from any one or more of the elements in the list of elements, but not necessarily including at least one of each and every element specifically listed within the list of elements and not excluding any combinations of elements in the list of elements. This definition also allows that elements may optionally be present other than the elements specifically identified within the list of elements to which the phrase "at least one" refers, whether related or unrelated to those elements specifically identified. Thus, as a non-limiting example, "at least one of A and B" (or, equivalently, "at least one of A or B," or, equivalently "at least one of A and/or B") can refer, in one embodiment, to at least one, optionally including more than one, A, with no B present (and optionally including elements other than B); in another embodiment, to at least one, optionally including more than one, B, with no A present (and optionally including elements other than A); in yet another embodiment, to at least one, optionally including more than one, A, and at least one, optionally including more than one, B (and optionally including other elements); etc.

It should also be understood that, unless clearly indicated to the contrary, in any methods claimed herein that include more than one step or act, the order of the steps or acts of the method is not necessarily limited to the order in which the steps or acts of the method are recited.

In the claims, as well as in the specification above, all transitional phrases such as "comprising," "including," "carrying," "having," "containing," "involving," "holding," "composed of," and the like are to be understood to be open-ended, i.e., to mean including but not limited to. Only the transitional phrases "consisting of" and "consisting essentially of" shall be closed or semi-closed transitional phrases, respectively, as set forth in the United States Patent Office Manual of Patent Examining Procedures, Section 2111.03.

Embodiments of the invention will now be described in the following numbered paragraphs:
1. A method of permitting a human or non-human primate subject to receive multiple doses of a recombinant adeno-associate virus (rAAV) vector, the method comprising:
   administering an anti-CD20 antibody and a subsequent rAAV vector to a human or non-human primate subject that has previously been administered a prior rAAV vector.
2. The method of paragraph 1, wherein the subsequent and prior rAAV vectors are the same serotype.
3. The method of paragraph 1 or 2, wherein the anti-CD20 antibody is administered more than once.
4. The method of any one of paragraphs 1 to 3, wherein the anti-CD20 antibody is administered at least once prior to the administration of the subsequent rAAV vector.
5. The method of any one of paragraphs 1 to 4, wherein the method further comprises administering the prior rAAV vector to the subject prior to the administration of the subsequent rAAV vector.
6. The method of paragraph 5, wherein the anti-CD20 antibody is administered more than once and wherein the anti-CD20 antibody is administered to the subject at least once prior to the administration of the subsequent rAAV vector and at least once prior to the administration of the prior rAAV vector.
7. The method of paragraph 1, wherein the method further comprises administering to the subject an mTOR pathway inhibitor.
8. The method of paragraph 7, wherein the mTOR pathway inhibitor is rapamycin.
9. The method of paragraph 7 or 8, wherein the anti-CD20 antibody and the mTOR pathway inhibitor are each administered more than once.
10. The method of any one of paragraphs 7 to 9, wherein the anti-CD20 antibody and the mTOR pathway inhibitor are each administered at least once prior to the administration of the subsequent rAAV vector.
11. The method of any one of paragraphs 7 to 10, wherein the method further comprises administering the prior rAAV vector to the subject prior to the administration of the subsequent rAAV vector.
12. The method of paragraph 11, wherein the anti-CD20 antibody and the mTOR pathway inhibitor are each administered more than once and wherein the anti-CD20 antibody and the mTOR pathway inhibitor are each administered to the subject at least once prior to the administration of the subsequent rAAV vector and at least once prior to the administration of the first rAAV vector.
13. The method of any one of paragraphs 1 to 12, wherein the anti-CD20 antibody is rituximab.
14. The method of any one of paragraphs 1 to 13, wherein the subject is a human.
15. The method of any one of paragraphs 1 to 14, wherein the administration of the prior rAAV vector to the subject occurs at least one day prior to the administration of the subsequent rAAV vector.
16. The method of paragraph 15, wherein the administration of the prior rAAV vector to the subject occurs at least one week prior to the administration of the subsequent rAAV vector.
17. The method of paragraph 15, wherein the administration of the prior rAAV vector to the subject occurs at least one month prior to the administration of the subsequent rAAV vector.
18. The method of paragraph 15, wherein the administration of the prior rAAV vector to the subject occurs at least one year prior to the administration of the subsequent rAAV vector.

## Claims

1. An anti-CD20 antibody for use in a method of preventing or reducing the immune response of a human subject to the administration of multiple doses of a recombinant adeno-associate virus (rAAV) vector, wherein the method comprises:
the administration of an anti-CD20 antibody and a subsequent rAAV vector to the human subject, wherein the human subject has previously been administered a prior rAAV vector,
wherein the method permits said human subject to receive multiple doses of said rAAV vectors.

2. The anti-CD20 antibody for use according to claim 1, wherein the subsequent and prior rAAV vectors are the same serotype.

3. The anti-CD20 antibody for use according to claim 1 or 2, wherein the anti-CD20 antibody is administered more than once.

4. The anti-CD20 antibody for use according to any one of claims 1 to 3, wherein the anti-CD20 antibody is administered at least once prior to the administration of the subsequent rAAV vector.

5. The anti-CD20 antibody for use according to any one of claims 1 to 4, wherein the method further comprises administering the prior rAAV vector to the subject prior to the administration of the subsequent rAAV vector;
optionally wherein the anti-CD20 antibody is administered more than once and wherein the anti-CD20 antibody is administered to the subject at least once prior to the administration of the subsequent rAAV vector and at least once prior to the administration of the prior rAAV vector.

6. The anti-CD20 antibody for use according to claim 1, wherein the method further comprises administering to the subject an mTOR pathway inhibitor.

7. The anti-CD20 antibody for use according to claim 6, wherein the mTOR pathway inhibitor is rapamycin.

8. The anti-CD20 antibody for use according to claim 6 or 7, wherein the anti-CD20 antibody and the mTOR pathway inhibitor are each administered more than once.

9. The anti-CD20 antibody for use according to any one of claims 6 to 8, wherein the anti-CD20 antibody and the mTOR pathway inhibitor are each administered at least once prior to the administration of the subsequent rAAV vector.

10. The anti-CD20 antibody for use according to any one of claims 6 to 9, wherein the method further comprises administering the prior rAAV vector to the subject prior to the administration of the subsequent rAAV vector.

11. The anti-CD20 antibody for use according to claim 10, wherein the anti-CD20 antibody and the mTOR pathway inhibitor are each administered more than once and wherein the anti-CD20 antibody and the mTOR pathway inhibitor are each administered to the subject at least once prior to the administration of the subsequent rAAV vector and at least once prior to the administration of the first rAAV vector.

12. The anti-CD20 antibody for use according to any one of claims 1 to 11, wherein the anti-CD20 antibody is rituximab.

13. The anti-CD20 antibody for use according to any one of claims 1 to 12, wherein the administration of the prior rAAV vector to the subject occurs at least one day prior to the administration of the subsequent rAAV vector; optionally wherein:
(i) the administration of the prior rAAV vector to the subject occurs at least one week prior to the administration of the subsequent rAAV vector;
(ii) the administration of the prior rAAV vector to the subject occurs at least one month prior to the administration of the subsequent rAAV vector; or
(iii) the administration of the prior rAAV vector to the subject occurs at least one year prior to the administration of the subsequent rAAV vector.

14. The anti-CD20 antibody for use according to any one of claims 1 to 13, wherein at least one of the prior rAAV vector and the subsequent rAAV vector comprises rAAV1, rAAV2, rAAV5, rAAV6, rAAV8, rAAV9, rAAV10, or AAVrh.10.
